# EUROPEAN PATENT SPECIFICATION

(11) **EP 2 131 790 B1**
(45) Date of publication and mention of the grant of the patent: **24.10.2012**
(21) Application number: 08725975.0
(22) Date of filing: 22.02.2008
(51) Int. Cl.: A61F 2/44, A61F 2/30, A61F 2/46, A61F 2/48

(54) **EXPANDABLE DEVICES FOR EMPLACEMENT IN BONE AND OTHER BODY PARTS**
EXPANDIERBARE VORRICHTUNGEN ZUR PLATZIERUNG IN KNOCHEN UND ANDEREN KÖRPERTEILEN
DISPOSITIFS EXPANSIBLES POUR POSITIONNEMENT DANS UN OS OU D'AUTRES PARTIES DU CORPS

(30) Priority: 22.02.2007 US 902668 P
(43) Date of publication of application: 16.12.2009
(73) Proprietor: Kyphon SÀRL, 2000 Neuchâtel (CH)
(72) Inventor: PHAN, Christopher, U., San Leandro, California 94578 (US)
(74) Representative: Viering, Jentschura & Partner
(86) International application number: PCT/US2008/002386
(87) International publication number: WO 2008/103466

(56) References cited:
- EP-A- 0 720 840
- WO-A-01/97721
- WO-A-03/073964
- WO-A-2008/011371
- DE-U1-202005 009 478
- FR-A- 2 774 280
- US-A1- 2004 186 569

## Description

### FIELD OF INVENTION

Embodiments of the present invention relate to expandable devices for emplacement in bone and other body parts.

### BACKGROUND

WO 03/073964 A1 discloses a distractible vertebral column implant comprising a first outer bushing, a second outer bushing coaxially arranged thereto, and an inner drive element that is screwed to at least one of the outer bushings. The inner drive element has a first thread, e.g. an external thread, whereby the outer bushing screwed to the drive element has a second thread, e.g. an internal thread, that fits with the external thread. The inner drive element rests against the supporting ring, and the drive element can be driven in the area of its face oriented toward the support ring.

Prosthetic devices may be used to repair a variety of body parts. For example, expandable devices such as fusion cages may provide a stabilized opening for inserting a bone graft between adjacent portions of bone. In time, the bone and bone graft can grow together through or around the fusion cage to fuse the graft and the bone solidly together. Current uses of fusion cages include treating a variety of spinal disorders, including degenerative disc diseases, Grade I or II spondylolistheses, adult scoliosis and other disorders of the lumbar spine. Spinal fusion cages are often inserted into the intervertebral disc space between two vertebrae for fusing them together. Such fusion cages can distract or expand a collapsed disc space between two vertebrae to stabilize the vertebrae by preventing the vertebrae from moving relative to each other.

Fusion cages may be used to connect any adjacent portions of bone or other solid body parts. However, one standard use of fusion cages is in the spine. Although often used in the lumbar spine, fusion cages can also be used in the cervical or thoracic spine. Insertion of a fusion cage into the spine is usually accomplished through a traditional open surgery which can be traumatic to the patient and require weeks, if not months, of recovery.

Corpectomy is an operation to remove a portion of the spine. Corpectomy may be performed to remove excess material (e.g., tumor, bone spurs, excess ligament growth) from a vetebral body so as to reduce compression of the spinal cord and spinal nerves. Or, corpectomy may be performed to remove a vertebral body. In some cases, an adjacent intervertebral disc may also be removed. Corpectomy may be done in the cervical (neck) region, the thoracic (chest) region, or the lumbar (lower back) region of the spine. Once the unwanted material has been removed, a bone graft, with or without an external support structure such as a metal plate or rod, may be used to reconstruct the spine and provide stability. For example, many patients undergoing corpectomy have tumor growths in the spine that needs to be removed. In this case, a physician may remove the vertebral body that contains the tumor growth as well as the adjacent discs.

In performing a corpectomy, the spine is usually accessed from the anterior side of the patient during the portion of the procedure where the spine is opened for removal of the vetebral body and discs, and an implant is inserted. In this way, the interior of the spine may be accessed without disturbing the spinal cord, which is located at the posterior side of the spine. However, if an external support structure is also required, the patient must then be accessed from the posterior side to position the support structure on the posterior side of the spine (and away from any internal organs). Most patients require an external support structure to prevent repositioning of the implant in the spine during normal movement of the back, and therefore, require surgical procedures on both the anterior and posterior sides of the body.

Accessing the spine from the posterior side of the patient is generally a much less invasive surgery than accessing the spine from the anterior side, as the only tissue that must be disturbed during posterior access is the skin and muscle of the back. Also, accessing the spine from the posterior may allow for the entire procedure to be done using only a single entry point, thereby significantly lessening the time required for patient recovery. However, because the spinal cord is on the posterior side of the spine, a posterior access must avoid injuring the spinal cord. A posterior access may therefore require that the implant that is being inserted into the spine have a small enough size so that the implant may be inserted into the center of the spine without disrupting the spinal cord.

Thus, there is a need to develop devices that allow for access to bone and other body parts where the access to the body structure is limited. For example, there is a need for devices that allow for corpectomy to be performed using a posterior access to thereby minimize patient recovery time. For a spinal corpectomy, such devices should if possible, allow for access to the spine via a mini-incision, but provide substantial support for the spine once implanted.

### SUMMARY

Embodiments of the present invention relate to expandable devices for emplacement in bone and other body parts. Embodiments of the present invention relate to corpectomy devices and methods of use. The present invention may be embodied in a variety of ways.

The invention provides an expandable device according to claim 1. Further embodiments of the invention are described in the dependent claims.

The contact element provides increased ability for the device to engage at least a portion of the body part into which the device is emplaced. For example, the contact element may be of a shape and size that is suitable for the surface of the contact element to engage a vertebral body or an intervertebral disc.

The present invention may comprise certain advantages. For example, the devices of the present invention may allow for corpectomy to be performed using only a posterior access route. Use of a single, posterior access route can significantly reduce recovery time (e.g., from greater than 6 months to about 3 months). Reducing the invasiveness of the procedure and the recovery time can substantially add to the quality of life for such subjects.

There are additional features of the invention which will be described hereinafter. It is to be understood that the invention is not limited in its application to the details set forth in the following description and figures.

### BRIEF DESCRIPTION OF THE FIGURES

FIG. 1 shows alternate embodiments (panels A-D) of expandable devices of the present invention before and after expansion of the device.
FIG. 2, panels A-C, shows a method for expanding an expandable device in accordance with one embodiment of the present invention.
FIG. 3 shows a perspective view of one embodiment of an expandable device of the present invention before expansion (panel A) and after partial expansion (panel B).
FIG. 4 shows a cross-sectional view of an expandable corpectomy device before , expansion (panel A) and after expansion (panel B) in accordance with one embodiment of the present invention.
FIG. 5 shows an exploded view of a expandable device in accordance with one embodiment of the present invention.
FIG. 6 shows a flowchart of a method for posterior corpectomy using an expandable corpectomy device in accordance with one embodiment of the present invention.
FIG. 7 shows an embodiment of a expandable device of the present invention emplaced between two adjacent level endplates.

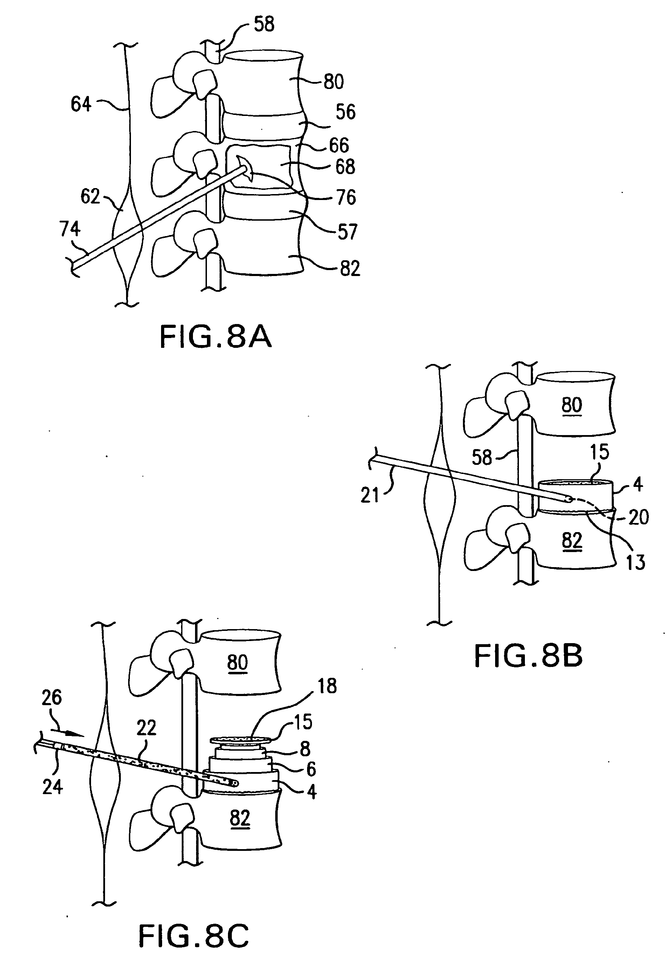

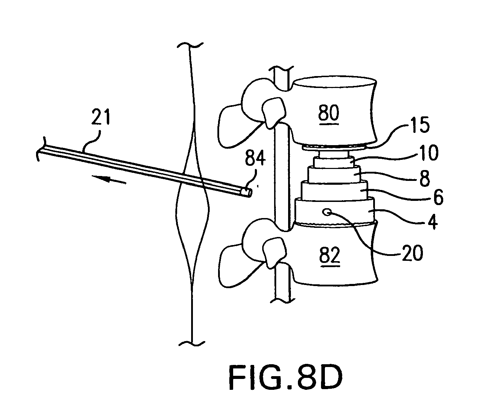

FIG. 8, panels A-D, shows a diagrammatic depiction of a procedure whereby a telescopic corpectomy device was implanted in a spine using a posterior approach in accordance with an embodiment of the present invention, where Panel A shows the spine with the vetebral body that is to be removed; panel B shows the telescopic corpectomy device being inserted into the spinal void without retraction of the cord; panel C shows the telescopic corpectomy device being expanded in the spinal void; and panel D shows the telescopic corpectomy device expanded in the spinal void and retraction of the access member.
FIG. 9 shows a kit comprising an expandable device in accordance with one embodiment of the present invention.

### DETAILED DESCRIPTION

Unless indicated to the contrary, the numerical parameters set forth in the following specification are approximations that can vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

It is further noted that, as used in this specification, the singular forms "a," "an," and "the" include plural referents unless expressly and unequivocally limited to one referent. The term "or" is used interchangeably with the term "and/or" unless the context clearly indicates otherwise.

Also, where ranges are provided, it is understood that other embodiments within the specified ranges are to be included.

As used herein, a subject is an animal. For example, the subject may comprise a mammal. In one embodiment, the subject may be a human. The user of the products, methods, and systems of the present invention may be a physician, veterinarian, a health care professional, or another person or device.

As used herein, an internal body part may comprise a structure in the body of a subject that may be accessed by an expandable device of the present invention. In some embodiments, the body part may comprise a bone, bony tissue (e.g., spinal tissue), or bones or part of a bone. The body part may comprise a portion of a spine, such as a vertebral body, an intervertebral spinal disc, or an intervertebral region. For example, due to various traumatic or pathologic conditions, such as cancer, a vertebral body or an intervertebral disc can experience fracture, degradation of the tissue, or expansion, and the like, that can lead to compression of the nerves, a reduction in mobility, and discomfort. The present invention is not, however, limited in application to the spine, spinal discs, vertebrae, or vertebral bodies, but may be used to repair other parts of a living or non-living organism. For example, in certain embodiments, the devices, methods, and systems or kits of the present invention can be deployed in other body structures, bones, or other tissue types, such as an arm bone, a leg bone, an organ, a portion of the vasculature, cartilage or tendons requiring surgical access and repair (e.g., a joint), and other body parts.

As used herein, an expandable device is a device that is emplaced within a body part or between adjacent body parts and expanded in at least one dimension. For example, the expandable device may be used during corpectomy to replace a vertebral body and/or vertebral disc(s). In certain embodiments, the expandable device may be a fusion cage that functions to provide support for two body parts such that fusion of the body parts may be performed. In certain embodiments, a fusion cage may be used to fuse two endplates together, as for example, when the disc between the two endplates has disintegrated. Or, the expandable device may be used to fuse two vertebral bodies together, as for example, when the discs and vetebral bodies between the two surrounding vertebral bodies has disintegrated. This is known as a corpectomy.

As used herein, an access path is an incision made in a subject to access an internal body part. The access path may be made, at least in part, using an access member.

As used herein, a percutaneous access is a procedure whereby access to an inner organ or tissue is done via needle puncture of the skin, rather than using an "open" approach where the inner organs or tissue are exposed (e.g., such as surgery or cutting the skin with a scalpel). For example, a percutaneous surgical access denotes passage through substantially unbroken skin, as for example, by needle puncture, a cannula or a catheter.

Also, as used herein, an access member comprises a device for accessing a predetermined location or body part in a subject. The inner volume of the access member may provide a path to access a region or a body part that is located within the subject's body. The access member may be any type of device that can extend from the location of interest (e.g., a bone or an organ) to be accessible to a user of the access member. For example, the access member may be designed to extend from an internal body part (e.g., a spine or other type of bone) in a subject to outside of the subject's body. Thus, an access member is a device that may be used by an operator (e.g., physician) to access the body part. As described in more detail herein, the access member may be an elongated hollow member such as a cylinder (solid or hollow), a tube, a cannula or a catheter. The access member may be inserted into the subject via a surgical incision made in the subject. In one embodiment, an access member is used to deliver the expandable device of the present invention to the body part. For example, the access member may comprise a rod or the like. In other embodiments, an access member may be used to deliver a material to the interior of the device. For example, the access member may comprise a hollow tube, such as a catheter or a cannula.

Also, as used herein, a material for emplacement within, or delivery to, a body part in a subject may comprise any material that is biologically compatible with the body part of interest. For example, in alternate embodiments, the material may comprise a bone filler material or an adhesive. As used herein, a bone filler material comprises any material that may be used for the replacement and/or treatment of bone tissue. A variety of materials have been described for use as bone filler materials (see e.g., U.S. Patent Nos. 4,904,257, 6,203,574, 6,579,532, 6,740,093, and Patent Publication No. 2005/0136038 for descriptions of bone filler materials). In one embodiment, the bone filler or treatment material may comprise PMMA. Alternatively, the bone filler material may comprise a cement, a gel, a fluid, or an adhesive, such as materials that are commercially available for repair of the spine and other bones or boney tissues. In other embodiments the bone graft material of the present invention may include allograft, autograft, BMP (bone-morphogenic proteins), bone marrow aspirate, demineralized bone matrix, ceramics, calcium phosphates, blood platelet gels, and other similar and known materials.

As used herein, the words "anterior" and "posterior" refer to the front and back of the subject, respectively.

As used herein, the term "member" refers to a part of an expandable device, where the members function together to form the expandable device, and were each member is similar in shape to each of the other members, but is not necessarily identical in size and/or shape to the other members.

As used herein, the term "contact element" is a part of an expandable device that functions to contact at least a portion of the body part into which the device is to be emplaced.

As used herein, the term "contact surface" is a surface of an expandable device that functions to contact at least a portion of the body part into which the device is to be emplaced.

### Expandable Devices

Embodiments of the present invention relate to expandable devices for emplacement in a predetermined location in a subject and methods of use of such devices. In certain embodiments, the predetermined location may comprise bone or another body part. The present invention may be embodied in a variety of ways.

In one embodiment, the present invention comprises an expandable device for emplacement in a body part, where the expandable device comprises a plurality of interlocking members that can assume a first unexpanded configuration where the plurality of members are substantially contained within the body of a first of the plurality of the members, and a second expanded configuration where the members are expanded along at least a first axis such that each of the members substantially emerge from the first member.

The device may, in certain embodiments also comprise a contact element that is suitable for engaging at least a portion of the body part. In certain embodiments, the contact element is attached to a member that is most distal to the first member when the device is in the expanded configuration. In one embodiment, the contact element may be entirely contained within the first member when the device is in the first unexpanded configuration. The contact element may be configured such that the surface of the contact element that is most distal to the first member has a greater surface area than the surface area of the surface of the member to which the contact element is attached. In this way, the contact element provides increased ability for the device to engage at least a portion of the body part into which the device is emplaced. For example, the contact element may be of a shape and size that is suitable for the surface of the contact element to engage a vertebral body.

Thus, as described herein, in certain embodiments, the an expandable device for emplacement in a bone or other body part may comprise (a) an expandable unit having a plurality of interlocking members; and (b) an upper surface and a lower surface functionally connected to the plurality of interlocking members. In an embodiment, at least one, or both, of the upper and lower surfaces comprises a contact element suitable for engaging at least a portion of the bone or other body part. Also in certain embodiments, the expandable device is capable of providing support to the bone or other body part in which it is emplaced. In one embodiment, the expandable device is capable of providing support to a spine of a subject when the device is implanted in the subject's spine during a corpectomy.

For example, in one embodiment, the present invention comprises an expandable device for emplacement in a body part, the expandable device comprising: (a) a plurality of interlocking members that can assume a first unexpanded configuration where the plurality of members are substantially contained within the body of a first of the plurality of the members, and a second expanded configuration where the members are expanded such that each of the members substantially emerges from at least one other member; and (b) a contact element that is attached to a member that is most distal to the first member when the device is in the expanded configuration, wherein the contact element is contained within the first member when the device is in the first unexpanded configuration, and wherein the contact element is configured such that the surface of the contact element that is most distal to the first member is suitable for engaging at least a portion of the body part, and wherein the surface of the contact element that is suitable for engaging at least a portion of the body part has a greater surface area than the surface area of the surface of the member to which the contact element is attached.

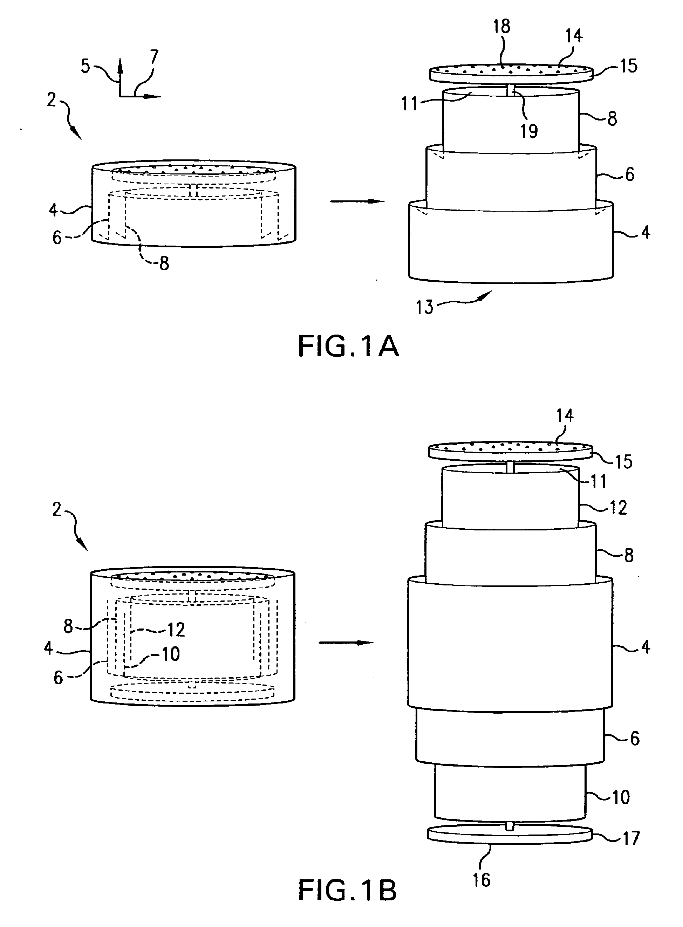

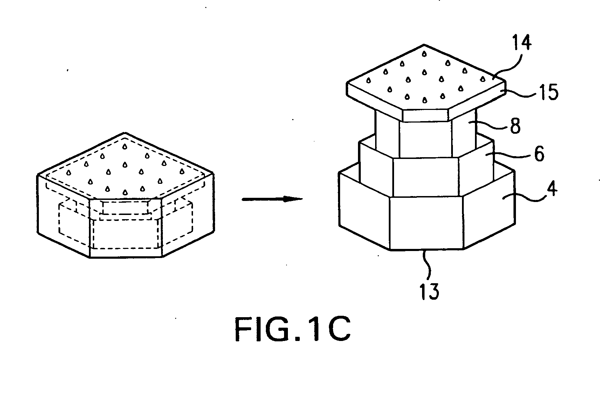

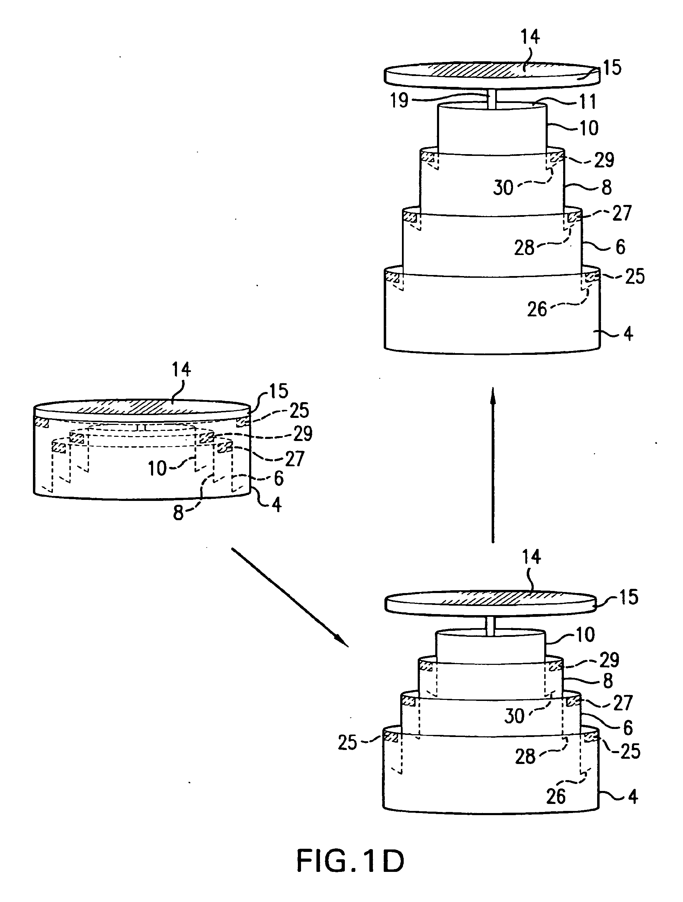

Embodiments of expandable devices are shown in FIG. 1. For example, as shown in FIG. 1A, an expandable device of the present invention 2, may comprise a plurality of members 4, 6, 8 that can assume a first unexpanded configuration (FIG. 1A, left side) or a second expanded configuration (FIG. 1A, right side). It can be seen, that in certain embodiments, the plurality of members 6, 8 are substantially contained within first member 4.

In certain embodiments, the axis along which the device expands 5 is substantially perpendicular to an axis that bisects the first member along its longest bisecting axis 7 or that is parallel to the base of the first member (see e.g., FIG. 1A). As used herein, a bisecting axis is an axis that bisects the member along at least one dimension. The longest bisecting axis is an axis that bisects the member along its longest dimension. For example, the device may comprise a plurality of members that expand out of a void in the first member along a single axis. The device may expand along an axis that is substantially perpendicular to the longest bisecting axis of the first member (see FIG. 1A). Or, the device maybe designed to expand along an axis that is not perpendicular to the base or the longest bisecting axis of the first member. For example, the members may be shaped such that upon expansion, the members expand along an axis that is at an angle to the base or the longest bisecting axis of the first member. This may occur where, for example, the side walls of the members are not equal in length around the entire perimeter of the member, such that upon expansion the members will lean in one direction. Or, the members may be positioned on a pivot, such that upon expansion, the members can be directed to expand out of the first member in a particular direction based on the positioning of the pivot point.

The individual members that make up the device may comprise a shape that allows for the members to attain a first unexpanded configuration and a second expanded configuration. In alternate embodiments, the members may be cylindrical, ovoid, substantially rectangular, rectangular with rounded sides, triangular, spherical, or any variety of shapes. For example, FIGS. 1A and 1B shows an expandable device with members that are substantially cylindrical, while FIG. 1C shows an expandable device with members that are five-sided. Other shapes that may be apparent to those of skill in the art are included in the expandable devices of the present invention.

Thus, the device may comprise any geometry that allows for the members to attain a first unexpanded configuration and a second expanded configuration. In certain embodiments, the plurality of members may be symmetrically arranged around a first axis (e.g., 5) such that the first member is the outer-most member. In one embodiment, the plurality of members are arranged concentrically around the first axis such that the first member is the outer-most member.

The members may comprise a shape that allows for a plurality of members to each be inserted into one of the other members. For example, in some embodiments, the device comprises a first outer member and a second member, where the second member has substantially the same shape as the first member but has a smaller perimeter than the first member, such that the second member may be inserted into the first member. The device may further comprise a third member, such that the third member has substantially the same shape as the second member but has a smaller perimeter than the second member such that the third member can be inserted into the second member. The device may further comprise a fourth member, such that the fourth member has substantially the same shape as the third member but has a smaller perimeter than the third member such that the fourth member can be inserted into the third member. Additional members, each having a substantially the same shape but smaller perimeter than another member may be included up to the smallest member which is inserted into the second smallest member.

In some embodiments, the device expands by a telescopic mechanism. For example, where the device has four members, as described above, such that the fourth member fits into the third member, which fits into the second member, which fits into the first outer member, the device may be expanded from a configuration where all the members are inserted in the first member, to an expanded configuration where the second member is expanded out of the first member, the third member is expanded out of the second member, and the fourth member is expanded out of the third member. Examples of devices expanding by such a telescopic mechanism are shown in FIG. 1A and 1C, where third member 8 expands out of second member 6 which expands out of first member 4. In a bilateral expansion as shown in FIG. 1B, members 10 and 12 may expand out of members 6 and 8 which expand out of member 4. Thus, in one embodiment, and as shown in FIG. 1A, the members comprise cylinders positioned concentrically around the first axis such that the first member is the outer-most member and when expanded the members comprise a plurality of tiered cylinders.

The device may expand in a single direction along a first axis 5 as shown in FIGS. 1A and 1C. Alternatively, the device may expand in two directions along the first axis 5 as shown in FIG. 1B. In other embodiments, the device may expand along a plurality of axes. For example, the device may comprise two sets of interlocking members that can expand along two different axes out of the top of outer first member 4 to make an expanded device that has a "V-shaped" configuration. Or, as discussed above, the members may be positioned on a pivot, such that upon expansion, the members can be directed to expand out of the first member in a particular direction based on the positioning of the pivot point.

In certain embodiments, the device is designed to engage a body part to thereby support that body part. Thus, in some embodiments, the device comprises at least one exterior surface that is suitable for engaging at least a portion of a body part into which the device is to be emplaced. Such surfaces 13 may be the surfaces of the individual members as shown in FIG. 1A and 1C. Or such surfaces may be a separate part comprising a contact element 15 that is attached via a connector 19 to one of the members 8 (see e.g., FIG. 1A). Thus, in one embodiment, the expandable device may comprise a contact element that is suitable for engaging at least a portion of the body part. In certain embodiments, the contact element is attached to a member that is most distal to the first member when the device is in the expanded configuration.

The contact element may be configured such that the surface of the contact element 14 that is most distal to the first member has a greater surface area than the area of the surface 11 of the member 8 to which the contact element 15 is attached (see e.g., FIG. 1A-1C). In this way, the contact element can provide increased ability for the device to engage at least a portion of the body part into which the device is emplaced. For example, the contact element may be of a shape and size that is suitable for the surface of the contact element to engage at least a portion of a vertebral body or an intervertebral disc upon emplacement and expansion of the device in a spine.

In one embodiment, the contact element 15 may be entirely contained within the first member 4 when the device is in the first unexpanded configuration (see e.g., FIGS 1A-1C). Or, the contact element may be positioned to provide a top or bottom surface (e.g., lid) to the first member (see e.g., FIG. 1D).

FIGS. 1A-1D show expandable devices that have separate contact elements 15, 17 that are attached to one of the interior members and that provide surfaces 14, 16 for engaging at least a portion of a body part (e.g., a vertebral body endplate). In certain embodiments, the surface 14 may comprise a roughened surface. For example, the surface may comprise ribbing, or a plurality of protrusions 18 as described in more detail herein (FIG. 1A-1C). Or, in some cases a relatively smooth surface may be used (see e.g., FIG. 1D).

In certain embodiments, the contact element is shaped to maximize contact with at least one surface of the body part. Thus, the expandable device may be substantially cylindrical in shape. Also, the upper and/or lower surfaces of the expandable device and/or the contact elements may be substantially circular in shape. For example, a cylindrical expandable device having a substantially circular lower surface and a circular upper surface on the contact element may be used where the device is employed for corpectomy and thus, is to be inserted in a substantially cylindrical void in the spinal cord. Other shapes may be used depending upon the void to be filled. For example, the device, and/or the upper or lower surfaces, may have a "kidney bean" shape to mimic the shape of a vertebral body. Or, the device, and/or the upper and lower surfaces, may, in alternate embodiments, be at least partly rectangular, cubic, hexagonal, octagonal, or have other polygonal shapes. In an embodiment, the shape of the upper and lower surface are the same as the openings at the upper and lower end of the first member such that when the unit is unexpanded (i.e., not deployed) the device is a substantially closed container.

Also, in alternate embodiments, the upper and lower external surfaces may be substantially flat, concave, or convex. The shape of the upper and lower surfaces may depend upon the use of the device, such that the device may be designed to tightly abut and/or support any body structures that are adjacent to the device after implantation and expansion. For example, for corpectomy, a convex surface may be used where the device abuts an endplate of a vertebral body.

In certain embodiments, the expandable device comprises an outer member that functions as a housing that contains the expandable device. In certain embodiments, the housing is the first outer member. In alternative embodiments, and as shown in FIG. 1D, the expandable portion of the device (i.e., members 4, 6, 8, 10) may fit into the housing, with the contact element 15 as an outer piece, thereby providing a lid. Or, the contact element may not necessarily close off one end of the first member, but may be sized such that the circumference of the contact element is less than the circumference of the first member. For example, in one embodiment, the contact element may not necessarily fit within the body of the first outer member when the device is in the unexpanded state, but may only cover a portion of the top opening of the outer member.

In an embodiment, the lower surface of the device may comprise the bottom surface 13 of the outer member (e.g., the bottom surface of the housing). For example, in one embodiment, the present invention may comprise an expandable device for emplacement in a bone or other body part that comprises: (a) a housing having sides and a lower surface; (b) an expandable unit that fits within the housing; and (c) a upper surface attached to the expandable unit. In one embodiment, when the device is expanded, the upper surface moves away from the lower surface. Thus, as described herein, in one embodiment, the members expand along an axis that is substantially perpendicular to the base or the longest bisecting axis (e.g., longitudinal axis) of the first outer member or housing.

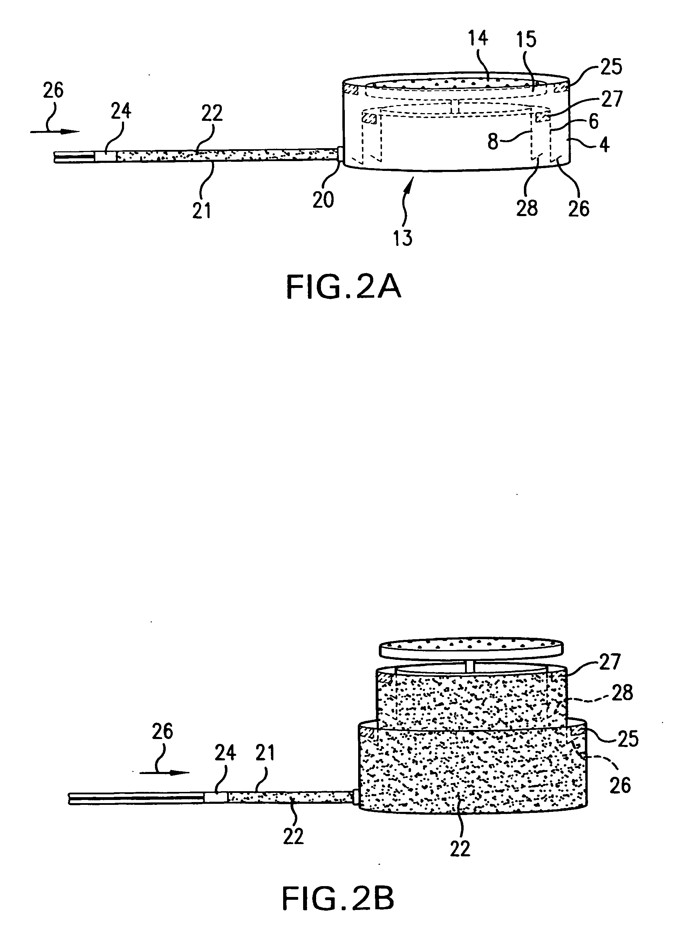

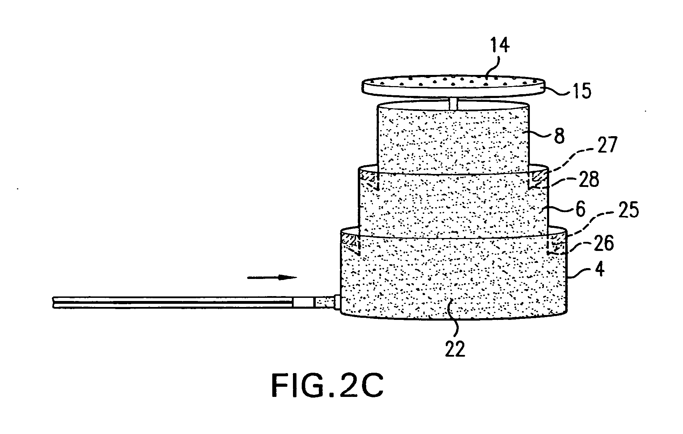

There may be a variety of methods used to expand the device. In some embodiments, a material is introduced into the device and is used to expand the device and to support the device in the second expanded configuration. FIG. 2 shows an example of a device of the present invention being expanded by introduction of a material 22 into the device. Thus, in certain embodiments, the device may comprise a first member that comprises an aperture 20 for insertion of a material into the interior of the device via a cannula or other type of access member 21. In alternate embodiments, the material may be a liquid or a semi-solid (e.g., viscous or paste-like). The material may also be a solid where the particles are small enough to fit in an aperture in the outer access member. For example, particles such as granulated bodies, miniature ball bearings or other small solid pieces may be used. Or, a mixture of liquid, semi-solid and solid may be used. For example, in some embodiments, the material may comprise a liquid bone cement in combination with particles and/or a paste-like (e.g., semi-solid) material.

In certain embodiments, the material used to expand the device may comprise a bone filler material. Thus, as shown in FIG. 2A, in certain embodiments, a material 22 may be introduced into the first member 4 via an aperture 20. For example, a plunger 24 may be used to deliver the material 22 into the expandable device via an access member 21 (e.g., cannula) by pushing the plunger towards 26 the aperture 20 in the expandable device. Or, a syringe having a cannula or catheter attached to the end may be used to introduce the material used to expand the device into the device. The material 22 may then fill the interior of member 4 thereby displacing member 6 (FIG. 2B) and also may fill the interior of member 6 thereby displacing member 8 (FIG. 2C). Additional members (not shown in FIG. 2) may be displaced (e.g., from larger to smaller) as the material is introduced into the member which contains the smaller member that will be displaced. The members may be displaced substantially sequentially, such that the second member 6 substantially emerges from the first member 4 prior to the third member 8 emerging from the second member 6 (see e.g., FIG. 2A-2C). Or, the members may be displaced substantially simultaneously, such that as member 6 begins to emerge from member 4, member 8 begins to emerge from member 6 and so forth (see e.g., FIG. 1D). Once expanded, the bone filler material and/or bone cement may harden, thereby fixing the device in its expanded configuration.

Or, other methods may be used to expand the device. For example, in certain embodiments, the device may comprise members that are connected by a spring mechanism. Or, a gas may be used to expand the device. In these embodiments that utilize a material that does not harden in the device once the device is expanded, the members may utilize latches, clips, or other mechanical mechanisms to maintain the expanded configuration.

There may be a variety of mechanisms by which the members are positioned in the expanded configuration. As discussed in more detail below, in some embodiments, at least some of the members comprise at least one retainer element that can engage at least a portion of another member to thereby limit the extent that the two members can move relative to each other. For example, in one embodiment, the members may comprise an upper retainer element that is able to engage a lower retainer element on at least one other member to thereby restrict the expansion of the two members with respect to each other and so, to restrict the overall expansion of the device. For example, where the device comprises a plurality of cylinders, at least some of the cylinders may comprise at least an upper retainer ledge 25, 27, 29 that can engage a lower retainer ledge 26, 28, 30 on another cylinder so as to restrict movement of the second cylinder with respect to the first cylinder (see e.g., FIG. 1D and 2A-C).

In certain embodiments, the expandable device may be used for corpectomy. For example, in certain embodiments, the expandable device comprises a corpectomy cage. Thus, in one embodiment, the expandable device is capable of providing support to a spine of a subject when the device is implanted in the subject's spine during a corpectomy. In some embodiments, the expandable device is suitable for use to replace a vertebral body and/or an intervertebral disc in a human spine. The expandable devices of the present invention may comprise a profile suitable for delivery via a percutaneous access, or by a mini-opening, or by a small incision. In an embodiment, the device has a profile suitable for inserting into the spine from the posterior of the subject during a corpectomy. In an embodiment, the device comprises a size prior to expansion that enables the device to be inserted into the spine during a posterior corpectomy.

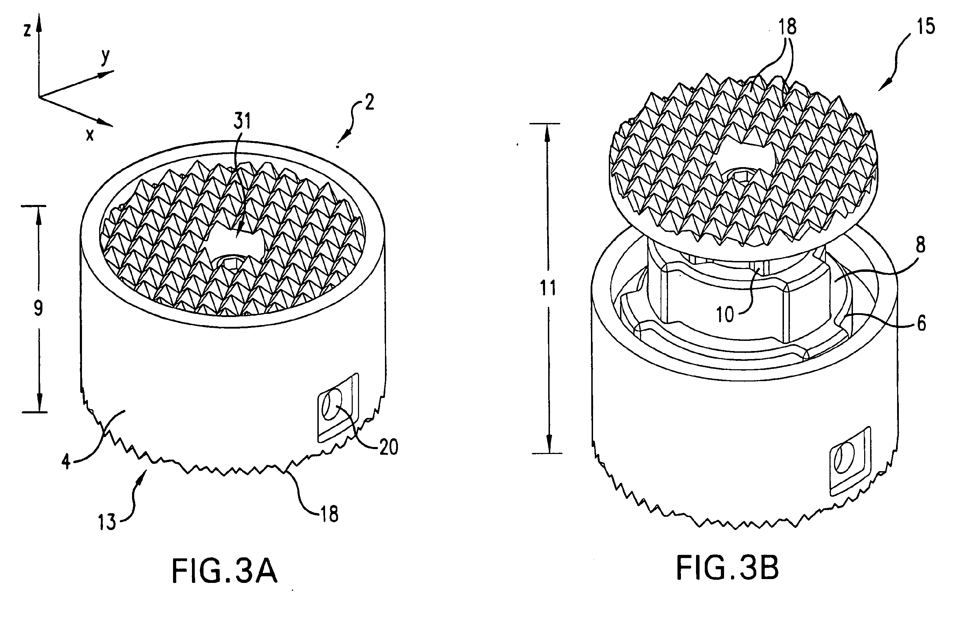

FIG. 3 shows a more detailed view of one embodiment of an expandable device 2 of the present invention. FIG. 3A shows the expandable device before deployment or expansion, and FIG. 3B shows the expandable device after partial deployment or expansion. It can be seen that the expandable device 2 may comprise a first member or housing 4 having a lower surface 13.

The device also may comprise inner members 6, 8, 10 that fit within the outer member (i.e., housing) 4. Also, in certain embodiments, the device may comprise an upper contact element 15 that is attached to one of the inner members 10.

In an embodiment, the upper contact element 15 may have a plurality of teeth or other protrusions 18. Also, the lower surface 13 of the housing or first member may also comprise teeth or other types of protrusions 18. As described herein, such teeth or other protrusions may facilitate emplacement of the device and stabilization of the device within the bone or other body structure. For example, when the device is emplaced in a spinal void, the protrusions 18 on the lower and upper surfaces may facilitate the device gripping to the endplate of an adjacent vertebral body or intervertebral disc that is above and/or below the void into which the device is emplaced. In some embodiments, the upper and/or lower surface(s) may comprise a notch 31 to allow for bone graft deposit. Also, in certain embodiments, the upper and/or lower surfaces may comprise a radioopaque marker, or other types of visually detectable chemical compounds, to allow the plates to be visualized by x-ray or other techniques. The use of a radioopaque compound or other types of markers as part of the upper and/or lower contact element may facilitate monitoring the emplacement of the device in the body part of interest. For example, X-ray imaging, MRI or other imaging techniques known in the art (e.g. fluoroscopy) may be used.

In one embodiment, the upper surface or contact element 15 may be designed such that it can be recessed within the outer most member or housing 4. For example, in alternate embodiments, the upper surface or contact element 15 may be recessed by about 0.1 to 5 mm, or from about 0.2 to 3 mm, or from about 0.3 to 2 mm, or about 0.5 to 1 mm. Or ranges within these ranges may be used. In this way, the protrusions (e.g., teeth) on the upper surface are not exposed to tissue during at least a portion of the implantation procedure.

In some embodiments, the outer member (or housing) 4 comprises an aperture or opening 20 for a filler material to be inserted into the housing or the expandable unit (see e.g., FIG. 3A). The filler material may comprise a bone filler or bone cement as described herein. The bone filler may be emplaced in the housing using an access member such as a cannula, catheter, or other type of emplacement mechanism.

The opening 20 may comprise a luer fitting, threads, pins or other type of quick connect mechanism for securing an access member such as a delivery tube or rod in the opening. The opening may be recessed as shown in FIG. 3, or may extend from the first member as shown in FIG. 2. The aperture and attached access member (e.g., cannula or catheter) may comprise a means by which the operator can manipulate the device into the body structure and/or to insert material (such as a bone filler or cement) into the interior of the device. In certain embodiments, it may be beneficial if the access member is made of a stiff material (e.g., surgical stainless steel) to allow for manipulation of the device and orientation in the spinal void. Also, in certain embodiments, it may be beneficial for the access member to be somewhat flexible to allow the device to be manipulated around other body parts as the device is guided to the body part into which the device is to be emplaced.

As described herein, in certain embodiments, the device may be designed such that the expandable members fit substantially in an outer member or housing. The expandable unit may be designed so as to expand in a direction that is substantially perpendicular to the base, or the longest bisecting axis (e.g., the longitudinal axis) of the housing. In one embodiment, the expandable unit is designed so as to expand substantially parallel to an external wall of the housing. Thus, the expandable unit may expand along the vertical axis (z-axis) of the device (FIG. 3) where the x and/or y axis are parallel to a longest dimension (e.g., the base, or the longest bisecting axis, or the longitudinal axis) of the unexpanded device.

The expandable device may, in certain embodiments, comprise a substantially closed container when in its unexpanded state. In one embodiment, the inner members of the expandable unit have a circumference that is less than the circumference of a housing into which the expandable members are emplaced.

In some embodiments, the expandable device may comprise a plurality of interconnected members. In an embodiment, the expandable structure may comprise a plurality of cylindrical units. For example, in an embodiment, the expandable structure may comprise a plurality of concentric cylindrical units, where the cylindrical units may be arranged in a manner to be concentric to each other and to the outer member or housing. Or, other embodiments that can provide a telescopic expansion may be used.

In some embodiments, the expandable unit comprises a plurality of tiered cylinders. The tiered cylinders may comprise at least one retainer element (e.g., ledge) that functions to restrict movement of a second cylinder along the first axis of the device. In an embodiment, positioning of the retainer ledges may be used to control the relative movement of the cylinders along the z-axis of the device, and thereby provide a limit the overall expansion of the expandable unit.

In certain embodiments, at least some of the tiered cylinders may have an upper retainer ledge that functions to restrict the motion of a second cylinder with respect to the first cylinder comprising the upper retainer ledge. Also, at least some of the tiered cylinders may have a lower retainer ledge that functions to restrict the motion of the first cylinder having the lower retainer ledge with respect to the housing or a another cylinder. In an embodiment the outer member or housing also has a retainer ledge positioned within the interior of the housing. The housing retainer ledge may be positioned so as to restrict the motion of the largest of the tiered cylinders along the first axis of the outer housing, i.e., so as to limit expansion.

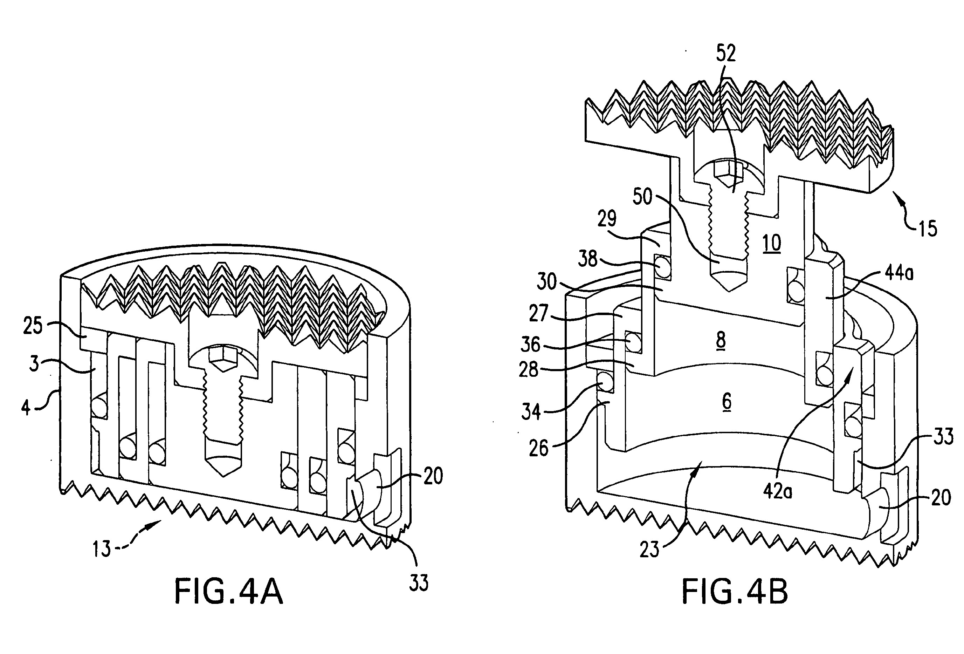

FIG. 4 shows an example of the deployment of an expandable device comprising a plurality of tiered cylinders having such retainer ledges in accordance with one embodiment of the present invention. Thus, in certain embodiments, the expandable unit may comprise an outer member 4 and a plurality of cylindrical units 6, 8, and 10. For example, as shown in FIG. 4, at least some of the cylindrical units 6, 8 may comprise an opening on one end (e.g., the lower end) of the cylinder. In an embodiment, the cylinders have different circumferences such that the plurality of cylinders fit inside each other to form a concentric array. For example, as shown in FIG. 4A, cylinder 10 fits inside of cylinder 8, cylinder 8 fits inside of cylinder 6, and cylinder 6 fits inside of the outer member or housing 4.

Still referring to FIG. 4, it may be seen that in certain embodiments, the cylinders comprise a plurality of interlocking retainer ledges. The retainer ledges may be directed towards either the interior or towards the exterior of the cylinder. In an embodiment, the upper retainer ledges 27, 29 point towards the central axis (e.g., the interior) of each member, and the lower retainer ledges 26, 28, 30 point towards the exterior of each member. The outer member or housing 4 may also have a retainer ledge 25. The housing retainer ledge may be positioned so as to restrict the motion of the first inner cylinder 6 (i.e., the second to largest member) with respect to the outer member (e.g., housing) 4. As discussed herein, in some embodiments, the retainer ledges only comprise a portion of the circumference of the cylinder.

For example, as illustrated in FIG. 4, the expandable unit may be expanded by the introduction of bone filler material or cement into a cavity 33 that is formed between the second to largest member 6 and the inner wall 3 of the first outer member or housing 4 (FIG. 4A). As the inner void 23 (of which cavity 33 is an outermost part) comprising the combined interior of each of the members 4, 6, 8 and 10 is filled with the bone filler material (or another substance), the first inner member 6 will be pushed upwardly (i.e., away from lower surface 13 of the outer member 4). Cylinder 6 will move upward until lower retainer 26 abuts the housing retainer ledge 25. In an embodiment, introduction of additional filler material will then push the next largest inner cylinder 8 upwardly, and fill the expanded void. Cylinder 8 will move upward until lower retainer 28 abuts the upper retainer ledge 27. Introduction of additional filler material will then push the innermost cylinder 10 upwardly. As noted herein, the members may be displaced substantially sequentially, for example, such that the second member 6 substantially emerges from the first member 4 prior to the third member 8 emerging from the second member 6. Or, the members may be displaced substantially simultaneously, such that as member 6 begins to emerge from member 4, member 8 begins to emerge from member 6 and so forth. It will be understood that substantially simultaneous displacement includes the embodiment where one member is displaced more than another member, but is not completely displaced before the beginning of the displacement of another member. For example, in a substantially simultaneous displacement of the device depicted in FIG. 4, it may be that the displacement of member 6 is greater than the displacement of member 8, which is greater than the displacement of member 10, and so forth. However, in substantially simultaneous displacement, at least two members are in the process of being displaced at the same time.

The innermost cylinder 10 can move upward until its lower retainer ledge 30 abuts the upper retainer ledge 29 of the cylinder 8 that encircles the innermost cylinder 10 such that expanded interior void 23 is filled with the bone filler or cement (FIG. 4B) (filler is not shown in FIG. 4). It will be understood that the sequence of expansion (i.e., deployment of the plurality of members of the expandable unit) may vary depending upon the friction between the members and the housing. In various embodiments, the frictional force may be varied by putting more or less interference between the interlocking parts (e.g., by varying size or material used for O-rings or other portions of the interlocking parts that abut each other).

In certain embodiments, the smallest member is solid. Or, the smallest member may be at least partially hollow. The configuration of the smallest member may depend on the materials used to make the members. Generally, the smallest member will comprise a strength sufficient to support a contact element attached to the member.

In one embodiment, O-rings or a similar cushioning 34, 36, 38 may be positioned between the upper and lower retainer ledges to prevent leaking of the bone filler or cement between the interlocking members (e.g., cylinders). It can be seen that when expanded, the expandable unit may resemble a stack of tiered cylinders.

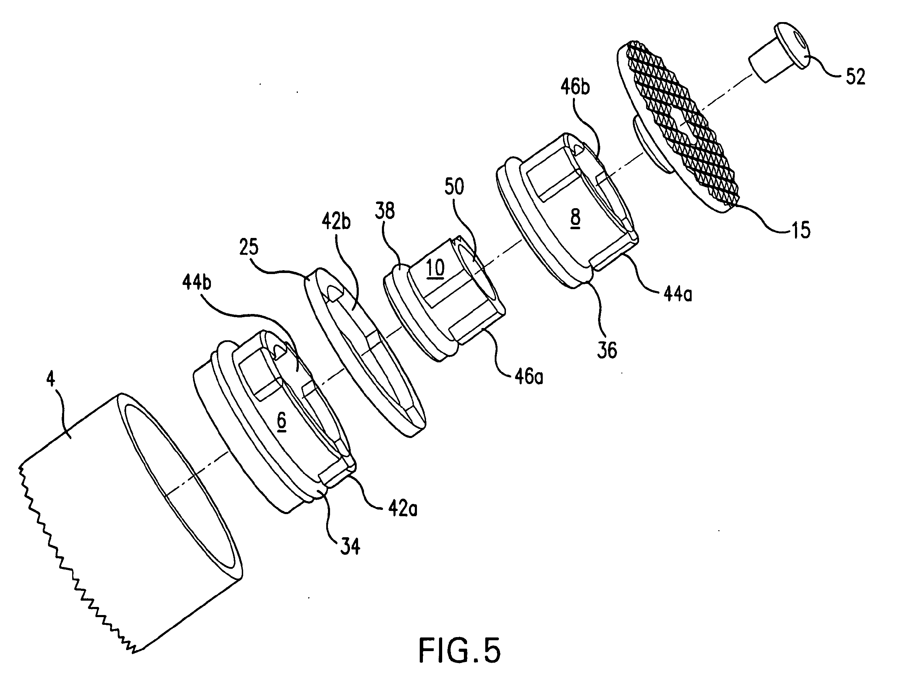

The plurality of interlocking members may comprise a means to prevent the members from slipping once the device is expanded. Thus, in certain embodiments, the plurality of interlocking members may comprise a means to prevent the members from slipping in the horizontal (x-y) direction upon expansion along the vertical (z) direction. In an embodiment, and as shown in FIGS. 4 and 5, the concentric cylinders may comprise a vertical ribbing e.g., 42a, 44a, 46a and matching wells 42b, 44b, 46b, respectively. For example, upon insertion of cylinder 8 into cylinder 6, rib 44a will fit into a well 44b in cylinder 6. Similarly, upon insertion of cylinder 6 into the housing 4, rib 42a will fit into a well in retainer 4. In an embodiment, the interlocking ribs and wells prevent the cylinders from rotating with respect to each other and the housing. Also, the ribs may be designed to overhang the O-rings (FIG. 5) and thus, can prevent the O-rings from slipping. Embodiments of the interlocking ribs 42a, 44a, 46a and wells 42b, 44b, 46b and the positioning of the ribs and O-rings are shown in FIG. 5 discussed below.

The expandable unit may have an upper contact element 15. For example, the innermost cylinder 10 may be attached to upper contact element 15. For example, innermost cylinder 10 may have an aperture 50 to allow the cylinder to be attached to upper surface piece 15 with a screw 52, expandable pin, or other attachment mechanism. Or, an adhesive may be used to attach the upper surface piece 15 to the innermost cylinder 10.

The device does not have to be expanded to its full height to be functional. In alternate embodiments, the expanded configuration comprises a height between the unexpanded configuration and a maximally expanded configuration. Thus illustrated in FIG. 4, when expanded, the entire device may have an extended length. The device of the present invention may be expanded to an extended height 11 that is at least 50%, or at least 100%, or at least 150%, or at least 200%, or at least 300%, or at least 400% longer than the starting length 9. Or, ranges within these ranges may be used, such that any level of expansion within these ranges may be obtained. For example, a device of the present invention may be expanded from about 12-15 mm in length to about 25-30 mm in length (a 2-fold or 100% expansion). Similarly, proportionally smaller or larger devices may be made (e.g., a device that expands from about 20-25 mm to about 40-50 mm, or any range of sizes in between). It is believed that this is generally a much greater expansion range than previous corpectomy devices, which generally may be expanded by about 25% to 33%.

The device provides flexibility in that a single device may be used for a variety of procedures as the expanded configuration comprises a height between the unexpanded configuration and a maximally expanded configuration. For example, for an expandable device that may expand from 15 mm to up to 30 mm, any level of expansion within this range (e.g., 16 mm, 17.2 mm, 27.4 mm, 29.8 mm) may be achieved. In this way, a single device may be used for corpectomy procedures at different places along the spine.

As discussed herein, in certain embodiments, the expandable device of the present invention may comprise a plurality of tiered cylinders. When the device is used for corpectomy, it may, in alternate embodiments, have a cross-sectional diameter (i.e., x-y direction) of about 15-35 mm, or 20-34 mm, or 24-32 mm, or about 30 mm depending upon the section of the spine undergoing repair. Or, ranges within these ranges may be used. For example, the diameter of a device used for a cervical corpectomy may be about 20 mm, whereas the diameter of a device used for a thoracic corpectomy may be about 30 mm, and the diameter of a device used for a lumbar corpectomy may be about 34 mm

In alternate embodiments, the number of members that make up the expandable unit may range from 1-10, or from 2-8, or from 2-6, or from 2-4 members. Or, ranges within these ranges may be used. For example, the telescoping members may be designed such that the diameter of the smallest member is about 20%, or 30%, or 40%, or 50%, or 60%, or 70%, or 75%, or 80%, or 85%, or 90% of the diameter of the largest member or housing. Or, ranges within these ranges may be used.

The thickness of the walls of each of the members may determine the total number of members that may be employed. In alternate embodiments, and depending upon the material used to make the device, the thickness of the walls of the members may range from about 0.5 mm to about 5 mm, or from about 1 mm to about 4 mm, or from about 2 mm to about 3 mm. Similar thickness ranges may also be used for the outer member or housing. Or, ranges within these ranges may be used.

As described in more detail herein, the device may be made of any material that is appropriate for use within a human or animal body. The materials used for the device may comprise materials that are typically used for prosthetic implants. Thus, in certain embodiments, the device may be made of a biocompatible plastic, such as, but not limited to polyetherketones (i.e., PEEK). Alternatively, the device may be made of a biocompatible metal, such as, but not limited to, stainless steel or titanium. Or combinations of such materials may be used. The device may, in certain embodiments, be made of a material that is compatible with the other parts of the system used for emplacement of the expandable device in a body part.

In certain embodiments, the device may be coated with any appropriate medical grade coating including an anti-infective, an anti-coagulant, a release coating, and/or a slipping agent. One of ordinary skill in the art having the benefit of this disclosure would appreciate that other materials, including those that are well-known to one in the art, may be applied to configure the access member described herein.

In some embodiments, a liquid or semi-solid material is used to expand the device. The filler used to expand the device may be a filler that is typically used to repair the bone or other body part of interest. For example, filler used for corpectomy may comprise polymethyl methacrylate (PMMA). Other types of bone filler material and/or bone cement that may be used include fillers as described herein, and/or fillers known for corpectomy and/or filling other body parts. Or, as described in more detail herein, a solid material may be used, where the particles are sized to fit in an aperture in the device so as to expand the device. Both the device and the filler may be made of materials that are approved by the FDA for implanting into humans.

In certain embodiments, the device and/or the cement material can be visualized using x-rays or other imaging techniques. For example, by using a filler material that is opaque to x-rays, the extent of repair of the spine may be monitored by magnetic resonance imaging (MRI) or other imaging techniques, not only during and immediately after surgery, but over the long-term.

### Methods of Making Expandable Devices

A method of making an expandable device for emplacing in a bone or other body part comprises fashioning an expandable device for emplacement in a body part, where the expandable device comprising a plurality of interlocking members that can assume a first unexpanded configuration such that the plurality of members are substantially contained within the body of a first of the plurality of the members, and a second expanded configuration wherein the members are expanded along at least a first axis such that each of the members substantially emerge from the first member.

The device may, in certain embodiments, also comprise at least one surface and/or contact element that is suitable for engaging at least a portion of the body part. In certain embodiments, method comprises fashioning a device having a contact element that is attached to a member that is most distal to the first member when the device is in the expanded configuration. In one embodiment, the contact element may be fashioned so as to be entirely contained within the first member when the device is in the first unexpanded configuration. Or, the contact element may be fashioned so as to provide a lid or other surface for the first member when the device is in the first unexpanded configuration. The contact element may be configured such that the surface of the contact element that is most distal to the first member has a greater surface area than the surface area of the surface of the member to which the contact element is attached. In this way, the contact element may provide increased ability for the device to engage at least a portion of the body part into which the device is emplaced. For example, the contact element may be fashioned to be of a shape and size that is suitable for the surface of the contact element to engage a vertebral body.

Another method to make an expandable device for emplacement in a body part provides an expandable device comprising: (a) a plurality of interlocking members that can assume a first unexpanded configuration wherein the plurality of members are substantially contained within the body of a first of the plurality of the members, and a second expanded configuration wherein the members are expanded such that each of the members substantially emerges from at least one other member; and (b) a contact element that is attached to a member that is most distal to the first member when the device is in the expanded configuration, wherein the contact element is contained within the first member when the device is in the first unexpanded configuration, and wherein the contact element is configured such that the surface of the contact element that is most distal to the first member is suitable for engaging at least a portion of the body part, and wherein the surface of the contact element that is suitable for engaging at least a portion of the body part has a greater surface area than the surface area of the surface of the member to which the contact element is attached.

For example the method of making an expandable device for emplacing in a bone or other body part may comprise: (a) fashioning an expandable unit having (i) a plurality of interlocking members and (ii) an upper surface and a lower surface functionally connected to the plurality of interlocking members, wherein at least one, or both, of the upper and lower surface are suitable for engaging at least a portion of the bone or other body part, and wherein the expandable device is capable of providing support to the bone or other body part in which it is emplaced; and (b) assembling the pieces to make an expandable device. In some embodiments, the present invention may comprise fashioning: (i) an outer housing having sides and a lower surface; (ii) an expandable unit that fits within the outer housing; and (iii) a upper contact element attached to the expandable unit; and assembling the pieces to make an expandable device. The expandable unit may, in some embodiments, comprise a plurality of tiered cylinders (or other units) such that the device expands by a telescopic mechanism.

FIG. 5 shows an exploded view of an expandable device in accordance with an embodiment of the present invention. FIG. 5 also illustrates how such a device having a first outer member or housing 4 with a closed bottom surface may be assembled. For example, in certain embodiments, the unit may be assembled by inserting the smaller of the interlocking members (e.g., 10) through the bottom of the next larger of the interlocking members (e.g., 8). For example, the third tier 10 with its O-ring 38 emplaced (and held in place by slipping the O-ring 38 under the ribbing 46a), may be inserted into the center of second tier 8 with its O-ring 36 emplaced, and then both tiers (10, 8) inserted into first tier 6 with its O-ring 34 emplaced.

Next, the assembly of tiered cylinders may then be inserted into the outer member or housing 4. The housing retainer 25 may then be emplaced within the interior of the housing 4 by positioning the retainer at the correct distance from the bottom surface 13 of the housing and affixing the retainer to the inner surface 3 of the housing 4 (see also FIG. 4). In one embodiment, the retainer 25 may be affixed to the inner surface 3 of the housing by an adhesive. Alternatively, the retainer 25 may be affixed to the inner surface 33 of the housing by heat sealing the two parts together. Or, the retainer 25 may be made of an expandable material such as a spring or the like that remains in position in the housing 4 once expanded. It can be seen that the ribs, e.g., 42a, 44a, 46a and associated wells, e.g., 42b, 44b, 46b may be used to align the cylinders with respect to each other and the housing.

Next, the upper contact element 15 may be attached to the smallest (i.e., the innermost) member 10. In an embodiment, a screw 52 or other fastener may be used to connect the upper contact element 15 to the innermost member 10 by inserting the screw into an aperture 50 on the upper surface of the innermost member 10. In an embodiment, a pivotable head may be used to attach the upper contact element 15 to the innermost member 10 of the expandable unit. In this way, the upper contact element may pivot to match the angle of the upper and lower endplates of the vetebral bodies that are adjacent to the device. Also, as described herein, in certain embodiments, the contact element may be shaped to mirror and/or to maximize contact with at least a portion of the body part into which the device is inserted. For example, contact element 15 and/or lower surface 13 as shown in FIG. 5 may be molded to be slightly convex and/or kidney shaped so as to abut an endplate of a vertebral body.

The individual parts of the device made of any material that is appropriate for use within a human or animal body. The materials used for the device may comprise materials that are typically used for prosthetic implants.

Thus, in certain embodiments, the individual members (e.g., 4, 6, 8, 10) may be made of a biocompatible plastic, such as, but not limited to polyetherketones (i.e., PEEK). Or, the device may be made of plastic, such as polypropylene, polyethylene, polyethyleneteraphthalate (PET), TEFLON®, ionomer, polycarbonate or nylon. Alternatively, the individual members may be made of a biocompatible metal, such as, but not limited to, stainless steel or titanium. In an alternate embodiment, the members may be made of NITINOL (i.e., a nickel titanium alloy). It should be understood that other materials are contemplated such as ultra high molecular weight polyethylene, silicone, and other metal materials, tantalum, platinum, niobium alloys, PHYNOX®, or any of a plurality of polymeric materials, such as polytetrafluoroethylene (ePTFE). Alternatively, the members may be made of any one of tungsten, iridium platinum, ELGILOY (Elgin, Illinois), or mixtures thereof or any other available metal alloy suitable for its intended purpose. Or, the device may be made of silicates or liquid crystal polymers.

Or combinations of such materials may be used. The expandable device may, in certain embodiments, be made of a material that is compatible with the other parts of the system. The O-rings may be made from a rubber or other polymer that is typically used for O-ring material in a device for implantation in a human subject. For example, O-rings made of silicone may be used.

One of ordinary skill in the art having the benefit of this disclosure would appreciate that other materials, including those that are well-known to one in the art, may be applied to configure the access member described herein.

Other materials/chemicals may be associated with the expandable device of the present invention such as lubricants, antibiotics, anti-cancer agents, bone cements, bone grafts, chemicals that prevent autoimmune defenses, and the like. In certain embodiments, the device may be coated with any appropriate medical grade coating including an anti-infective, an anti-coagulant, a release coating, and/or a slipping agent. In an embodiment, these materials/ chemicals may be released over an extended time period. These materials/chemicals may be used to aid accretion of bone or of tissue, to aid in fighting off bacterial infections, to promote tissue growth or for a plurality of other reasons. Such agents may be coated onto the expandable device, or attached as time-release capsules or by other means known in the art.

The device does not have to be expanded to its full height to be functional. In alternate embodiments, the expanded configuration comprises a height between the unexpanded configuration and a maximally expanded configuration. Thus illustrated in FIG. 4, when expanded, the entire device may have an extended length. The device of the present invention may be expanded to an extended height 11 that is at least 50%, or at least 100%, or at least 150%, or at least 200%, or at least 300%, or at least 400% longer than the starting length 9. Or, ranges within these ranges may be used, such that any level of expansion within these ranges may be obtained. For example, a device of the present invention may be expanded from about 12-15 mm in length to about 25-30 mm in length (a 2-fold or 100% expansion). Similarly, proportionally smaller or larger devices may be made (e.g., a device that expands from about 20-25 mm to about 40-50 mm, or any range of sizes in between). It is believed that this is generally a much greater expansion range than previous corpectomy devices, which generally may be expanded by about 25% to 33%.

The device provides flexibility in that a single device may be used for a variety of procedures as the expanded configuration comprises a height between the unexpanded configuration and a maximally expanded configuration. For example, for an expandable device that may expand from 15 mm to up to 30 mm, any level of expansion within this range (e.g., 16 mm, 17.2 mm, 27.4 mm, 29.8 mm) may be achieved. In this way, a single device may be used for corpectomy procedures at different places along the spine.

As discussed herein, in certain embodiments, the expandable device of the present invention may comprise a plurality of tiered cylinders. When the device is used for corpectomy, it may, in alternate embodiments, have a cross-sectional diameter (i.e., x-y direction) of about 15-35 mm, or 20-34 mm, or 24-32 mm, or about 30 mm depending upon the section of the spine undergoing repair. Or, as discussed above, ranges within these ranges may be used. For example, the diameter of a device used for a cervical corpectomy may be about 20 mm, whereas the diameter of a device used for a thoracic corpectomy may be about 30 mm, and the diameter of a device used for a lumbar corpectomy may be about 34 mm.

For example, the telescoping members may be designed such that the diameter of the smallest member is about 20%, or 30%, or 40%, or 50%, or 60%, or 70%, or 75%, or 80%, or 85%, or 90% of the diameter of the largest member or housing. Or, ranges within these ranges may be used.

The thickness of the walls of each of the members may determine the total number of members that may be employed. In alternate embodiments, and depending upon the material used to make the device, the thickness of the walls of the members may range from about 0.5 mm to about 5 mm, or from about 1 mm to about 4 mm, or from about 2 mm to about 3 mm. Similar thickness ranges may also be used for the outer member or housing. For example, in an embodiment where the wall thickness of the members is about 2-3 mm, an expandable unit used for corpectomy may comprise about 3 members. Or, ranges within these ranges may be used.

For example, where the device is used for corpectomy and comprises 3 cylinders and each cylinder has walls about 2.0 mm thick, the housing may be about 30 mm in diameter and the smallest cylinder may be about 12 mm in diameter, with the cylinders in between being proportionately sized (e.g., about 18 and 24 mm, respectively, in diameter) relative to the housing and smallest cylinder. Other expandable devices having similar dimensions suitable for expansion in the spine are included in the present invention.

### Method of Emplacing an Expandable Device

In a method for inserting an expandable device into a spine or other body structure where the access to the body structure is limited, and therefore requires that the device be minimized in size during the procedure for delivering the device to the body part. The expandable device may comprise a profile suitable for delivery by a mini-opening, or by a small incision. Or in some cases, a percutaneous access may be used. or inserting an expandable device from the posterior of the subject during a corpectomy.

The expandable device may comprise a plurality interlocking members that can assume a first unexpanded configuration where the plurality of members are substantially contained within the body of a first of the plurality of the members, and a second expanded configuration where the members are expanded such that each of the members substantially emerges from at least one other member. The device may further comprise a contact element that is attached to a member that is most distal to the first member when the device is in the expanded configuration and that provides a surface that is suitable for engaging at least a portion of the body part when the device is expanded. The method may comprise the step of expanding the device *in situ* (i.e., in the body part) by the introduction of a filler, such as a bone repair material or bone cement, into the device.

The method for inserting an expandable device from the posterior of the subject for a posterior corpectomy may comprise: (a) creating a void in the subject's spine via the access created by a posterior incision; (b) inserting an expandable device of into the spinal void; and (c) expanding the device.

The method for inserting an expandable device from the posterior of the subject for a posterior corpectomy may comprise: (a) creating a void in the subject's spine via the access created by the posterior incision; (b) inserting an expandable device into the spinal void, wherein the expandable device comprises (i) an expandable unit having a plurality of interlocking members that can assume a first unexpanded configuration where the plurality of members are substantially contained within the body of a first of the plurality of the members, and a second expanded configuration where the members are expanded along a first axis such that each of the members substantially emerge from the first member; and (c) expanding the device by the introduction of a filler material into the device. In one embodiment, the expandable device comprises an upper surface and a lower surface functionally connected to the plurality of interlocking members, wherein at least one, or both, of the upper and lower surface are suitable for engaging at least a portion of the bone or other body part.

In certain embodiments, the device may comprise a contact element that is suitable for engaging at least a portion of the body part. For example, in some embodiments the present invention may comprise a method for inserting an expandable device from the posterior of the subject for a posterior corpectomy comprising: (a) creating a void in the subject's spine via the access created by a posterior incision; (b) inserting an expandable device of into the spinal void, wherein the expandable device comprises (i) a plurality of interlocking members that can assume a first unexpanded configuration where the plurality of members are substantially contained within the body of a first of the plurality of the members, and a second expanded configuration where the members are expanded along a first axis such that each of the members substantially emerge from the first member, and (ii) a contact element that is attached to a member that is most distal to the first member when the device is in the expanded configuration, wherein the contact element is contained within the first member when the device is in the first unexpanded configuration, and wherein the contact element is configured such that the surface of the contact element that is most distal to the first member is suitable for engaging at least a portion of the body part, and wherein the surface of the contact element that is suitable for engaging at least a portion of the body part has a greater surface area than the surface area of the surface of the member to which the contact element is attached; and (c) expanding the device. In an embodiment, the contact element is contained substantially completely in the first member prior to expansion of the device. Or, the contact element may comprise an outer surface of the unexpanded device. For example, the contact element may comprise a top surface or lid for an unexpanded device as shown in FIG. 1D.

The expandable device may be capable of providing support to the bone or other body part in which it is emplaced. In one embodiment, the expandable device is capable of providing support to a spine of a subject when the device is implanted in the subject's spine during a corpectomy. Also, the device may, in certain embodiments, comprise a size prior to expansion that enables the device to be inserted into the spine during a posterior corpectomy.

In some embodiments, the device may comprise an outer member that functions as a housing into which the additional members of the expandable unit are emplaced. In an embodiment, the lower surface of the device is part of the housing. For example, in certain embodiments, the expandable device for emplacement in a bone or other body part comprises: (a) a housing having sides and a lower surface; (b) an expandable unit comprising a plurality of inner members that fit within the housing; and (c) a upper surface attached to at least one of the plurality of inner members.

For example, when the device is expanded, the upper surface moves away from the lower surface. In one example embodiment, the expandable unit may comprise a plurality of tiered cylinders.

Also, in some embodiments, at least one of the upper or lower surfaces and/or contact elements of the device is retracted into the device during insertion of the device into the body part needing repair, so the surface cannot come into contact with, an perhaps cause damage to, tissue in the body part. Where the expandable device is used for corpectomy, the device may, in certain embodiments, comprise an upper contact element that is retracted into the outermost member or housing. Once the device has been inserted into the spine, it may be expanded along the length of the spine, thereby allowing any protrusions that are present on the upper contact element to emerge from the outer member or housing so as to engage an adjacent disc or the endplate of a vetebral body.

The housing or the outermost member of the expandable device may comprise an aperture or opening for a filler material to be inserted into the housing and/or expandable unit. For example, the opening may comprise a luer fitting, threads, pins or other type of quick connect mechanism for securing an access member such as a delivery tube, cannula or catheter in the opening. In this way, the aperture and access member may comprise a means by which the operator manipulates the device into the bone or other body structure.

To expand the device, a filler or other type of material may be injected into the device. The filler material may comprise a bone filler or bone cement as described herein (e.g., PMMA). For example, a bone filler material may be emplaced in the housing using an access member such as a catheter or other type of tubular emplacement mechanism to thereby expand the expandable device as shown for example, in the embodiments of FIG. 2.

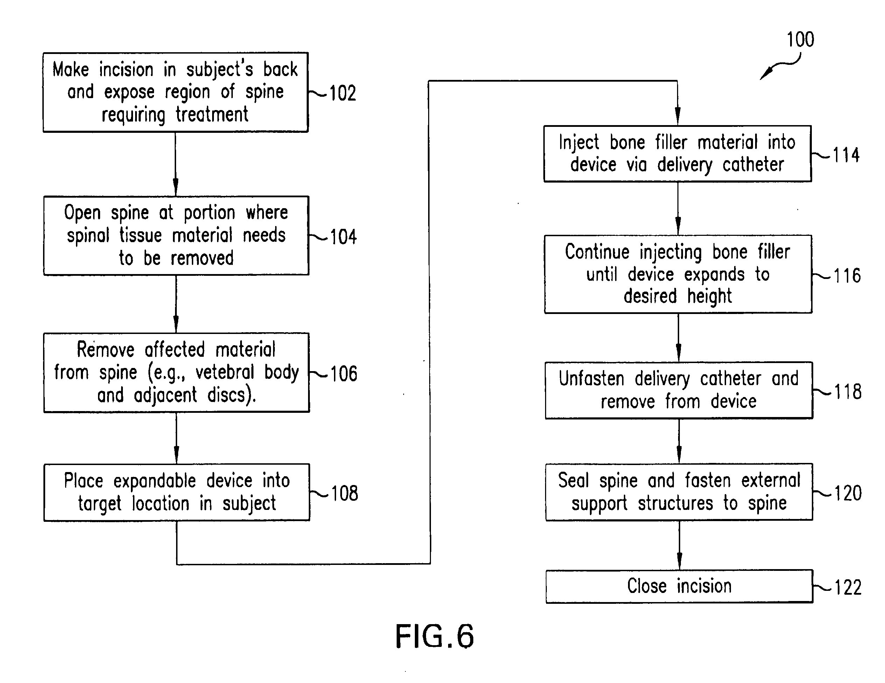

FIG. 6 shows a method 100 for posterior corpectomy using an expandable device of the present invention. For example, in some embodiments of the method of FIG. 6, the expandable device is a telescopic corpectomy cage. Thus, in one embodiment, a midline incision can be made in the subject's back and the region of the spine requiring repair (e.g., corpectomy) is exposed 102. Next, the portion of the spine comprising the affected area is opened 104, and the diseased tissue (e.g., tumor, spurs, excess ligament tissue) is removed 106. In an embodiment, the entire vetebral body and adjacent disc(s) are removed.

At this point, an expandable device of the invention may be inserted into the incision in the subject's back 108 and then manipulated using an access member such as a substantially stiff rod, a catheter, or by other means so that it is in position to be inserted into the spinal void.

The device may then be positioned in the spine such that at least one contact surface (e.g., a retracted contact element) is oriented towards one end (e.g., the top) of the spine and another contact surface (e.g., the non-retracted bottom surface of outer member 4) is oriented towards the other end (e.g., bottom) of the spine. Then, a filler material may be injected into the device 114. The filler material may be injected until the device has been expanded to the desired height along the axis that is generally parallel to the spine 116. In one embodiment, the device is expanded until the upper surface and the lower surface of the device abut the portion of the spine that is adjacent to the void. For example, the upper and lower surfaces of the device may abut the neighboring discs and/or the end-plate of a neighboring vetebral body.

Once the device has been expanded to the desired amount, the access member for delivery of the bone filler material may be unfastened from the device and removed from the subject 118. The opening in the spine may then be sealed (e.g., with a metal plate), and any external supporting hardware (e.g., rod) that is required, may be attached to the posterior side of the spine 120. At this point, the incision may be closed 122.

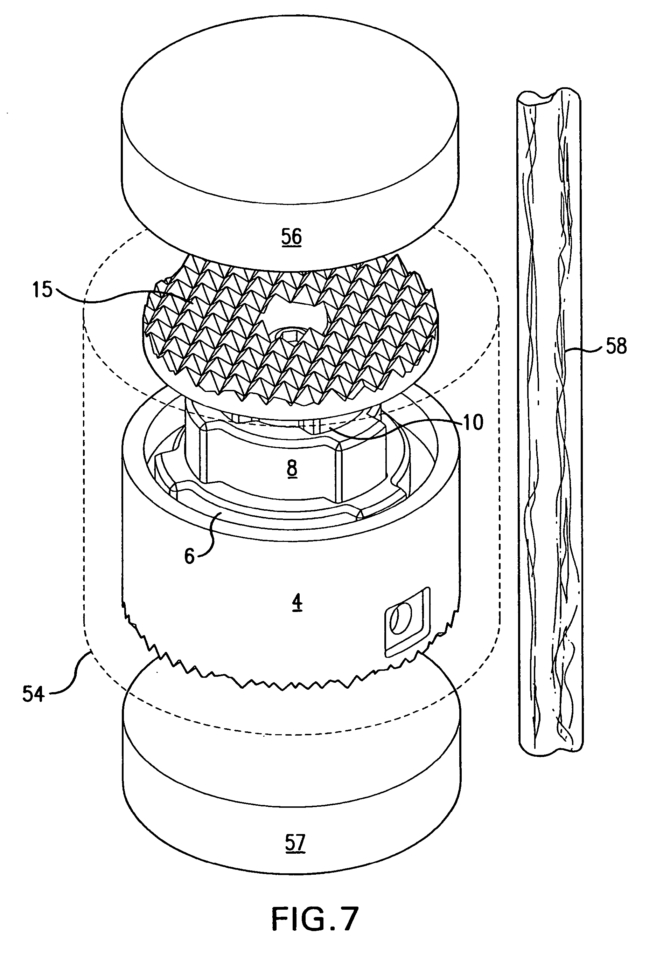

FIG. 7 illustrates insertion of an expandable device of the present invention into a void in a spine. It can be seen that because the void 54 in the spine is substantially cylindrical in shape, the expandable device 2 fits into the void. The upper surface piece of the device 15 may be retracted into the housing prior to emplacement in the subject's spine. In this way, the protrusions 18 on the upper surface of the device will not impinge upon the exposed spinal cord 58. Once the device is emplaced in the void, it may be expanded such that the upper surface 14, and the lower surface 13 can impinge on the endplates of the adjacent upper and lower vertebral bodies 56 and 57, respectively.

FIG. 8, panels A-D, shows depiction of a telescopic corpectomy device of the present invention being used during a posterior corpectomy procedure. FIG. 8A shows surgical access into the vertebral body via a posterior lateral approach. As shown in FIG. 8A an incision 62 may be made in the subject's back 64 to remove a vertebral body 66 containing a tumor 68 and adjoining discs 56, 57. For example, as shown in FIG. 8A, an access member 74 having a curette, knife, rongeur blade or the like 76 on the end may be used to access the diseased vertebral body. The diseased vertebral body and any adjacent structures as necessary may then be removed as is known in the art.

Figure 8B shows the delivery and insertion of a telescopic corpectomy device of the present invention in the spinal void without retraction of the cord 58 using access member 21. It can be seen that in one embodiment, at least one contact element 15 is retracted into the first member (housing 4) and so, any protrusions 18 (see e.g., FIG. 8C) on the contact element do not come into contact with the cord 58. In some embodiments, the protrusions on one side of the device comprise the bottom of the first member. In this case, the protrusions may be exposed during insertion of the device into the body part.

FIG. 8C shows the telescopic device being expanded inside of the spinal void by insertion of a bone filler 22 via a plunger 24 being pushed in direction 26 through the catheter 21. As the filler material enters the device, it can fill any voids within the individual members 4, 6, 8 such that member 6 is pushed out of member 4, and member 8 (to which the contact element 15 is attached) is pushed out of member 4 and member 6.

FIG. 8D shows the device expanded and filled with the bone filler material (22). It can be seen that the contact element of the device is in contact with the endplate of at least one adjacent vertebral body 80. The second contact surface 13, which in this case comprises the bottom surface of the housing 13, is in contact with the lower adjacent vertebral body 82. Also seen is the luer fitting 84 on the delivery catheter 21 which may be used to connect (and disconnect) the catheter from the expandable device. In this way, once the device is in position and expanded, the access member may be removed and the incision in the subject's back closed.

### Kits and Systems

In other embodiments, the expandable device may be part of a system or a kit. Thus, in other embodiments, the present invention comprises a kit or system comprising an expandable device for emplacement in a bone or other body part.

Any of the embodiments of the devices of the present invention as described herein may be used in the systems and kits of the present invention. In some embodiments the device used in the systems or kits of the present invention may comprise (a) an expandable unit having a plurality of interlocking members; and (b) an upper surface and a lower surface functionally connected to the plurality of interlocking members, wherein at least one, or both, of the upper and lower surfaces are suitable for engaging at least a portion of the bone or other body part, and wherein the expandable device is capable of providing support to the bone or other body part in which it is emplaced. The system and/or kits may further comprise an access member for emplacement of the expandable device and/or for insertion of a material into the expandable device to thereby expand the device.

In one embodiment, the systems or kits of the present invention comprise an expandable device for emplacement in a body part, where the expandable device comprises a plurality of interlocking members that can assume a first unexpanded configuration where the plurality of members are substantially contained within the body of a first of the plurality of the members, and a second expanded configuration where the members are expanded along at least a first axis such that each of the members substantially emerge from the first member.

The device of the systems or kits of the present invention may, in certain embodiments also comprise a contact element that is suitable for engaging at least a portion of the body part. In certain embodiments, the contact element is attached to a member that is most distal to the first member when the device is in the expanded configuration. In one embodiment, the contact element may be entirely contained within the first member when the device is in the first unexpanded configuration. The contact element may be configured such that the surface of the contact element that is most distal to the first member has a greater surface area than the surface area of the surface of the member to which the contact element is attached. In this way, the contact element provides increased ability for the device to engage at least a portion of the body part into which the device is emplaced. For example, the contact element may be of a shape and size that is suitable for the surface of the contact element to engage a vertebral body.

For example, in some embodiments, the present invention may comprise a kit or a system comprising: (a) an expandable device for emplacement in a body part in a subject, the expandable device comprising (i) a plurality of interlocking members that can assume a first unexpanded configuration where the plurality of members are substantially contained within the body of a first of the plurality of the members, and a second expanded configuration where the members are expanded such that each of the members substantially emerges from at least one other member, and (ii) a contact element that is attached to a member that is most distal to the first member when the device is in the expanded configuration, wherein the contact element is contained within the first member when the device is in the first unexpanded configuration, and wherein the contact element is configured such that the surface of the contact element that is most distal to the first member is suitable for engaging at least a portion of the body part, and wherein the surface of the contact element that is suitable for engaging at least a portion of the body part has a greater surface area than the surface area of the surface of the member to which the contact element is attached; and (b) an access member configured to be attached to the device and suitable for using to insert the device into the body part and/or for insertion of a material into the expandable device to thereby expand the device.

The expandable device of the kits or systems of the present invention may be capable of providing support to the bone or other body part in which it is emplaced. Also, the expandable devices of the kits or systems of the present invention may comprise a profile suitable for delivery via a percutaneous access, or by a mini-opening, or by a small incision. For example, in some embodiments, the expandable device is capable of providing support to a spine of a subject when the device is implanted in the subject's spine during a corpectomy. In certain embodiments, the expandable device of the kits or systems of the present invention may be used to replace a vertebral body and/or an intervertebral disc. In an embodiment, the device of the systems or kits of the present invention comprises a size prior to expansion that enables the device to be inserted into the spine during a posterior corpectomy.

The device of the systems or kits of the present invention may comprise a housing to surround the expandable unit. Thus, in certain embodiments, the expandable device may further comprise a first member or housing into which the expandable unit is emplaced. In one embodiment, the lower surface may comprise part of the housing. For example, in one embodiment, the present invention may comprise an expandable device for emplacement in a bone or other body part that comprises: (a) a housing having sides and a lower surface; (b) an expandable unit that fits within the housing; and (c) a upper surface attached to the expandable unit. In an embodiment, when the device is expanded, the upper surface moves away from the lower surface. For example, in an embodiment, the expandable unit comprises a plurality of tiered cylinders.

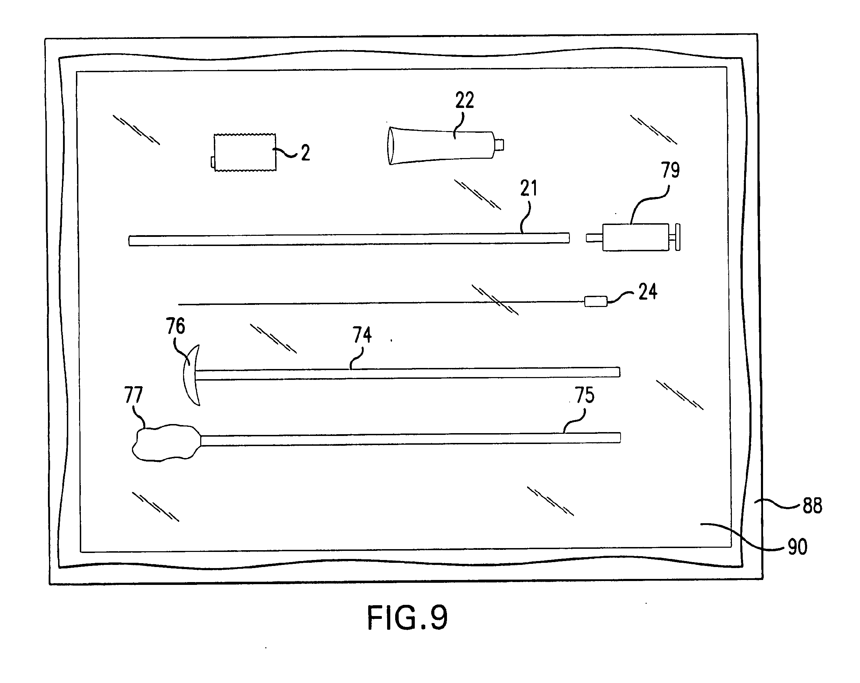

FIG. 9 shows an illustration of one embodiment of a kit of the present invention. Thus, as shown in FIG. 9, the kit may comprise one or more expandable devices 2 of the present invention having a plurality of interlocking members that can assume a first unexpanded configuration wherein the plurality of members are substantially contained within the body of a first of the plurality of the members, and a second expanded configuration wherein the members are expanded such that each of the members substantially emerges from at least one other member, and that has a contact element that is attached to a member that is most distal to the first member when the device is in the expanded configuration, wherein the contact element is contained within the first member when the device is in the first unexpanded configuration.

The kit may further comprise a pushing member 24 (e.g., plunger), or multiple pushing members for urging the expandable device through the cannula. Also, the kit may comprise a syringe for injecting a material (e.g., via an access member such as a cannula or a catheter) into the device. The kit and systems of the present invention may also include an access member 75 and inflatable members 77. For example, in certain embodiments, the kits and systems may be used to emplace an inflatable member into the body part to support the body part during emplacement of the expandable device. Also, a plurality of inflatable members may be included. In one embodiment, the inflatable members may comprise balloons, such as those used in kyphoplasty. Or, other inflatable members may be used. The kit may also include other tools needed for access of the body part as for example, access members 74 with a blade or curette 76 for removing portions of the body part as needed.

Embodiments of the systems and/or kits of the present invention may further comprise a material 22 to be delivered to the internal body part. For example, where the system is being used for bone repair, the access member, or a tube or syringe may be loaded with a bone filler material or an adhesive. In an embodiment, the bone filler material may comprise a mixture containing calcium, hydroxyl apatite, and a polymer. Also, in certain embodiments, the bone filler may comprise ceramic granules or other filler material. For corpectomy, the filler may be PMMA.

Expandable devices of the present invention may be designed for emplacement in a variety of body parts. For example, the expandable devices may be emplaced in a boney structure, or between two bones. In one embodiment, the expandable device may be emplaced between two vertebral bodies, to replace the intervertebral disc. In certain embodiments of the systems and kits of the present invention, the expandable device is configured for use to replace a vertebral body and/or an intervertebral disc in spinal corpectomy via a posterior access. The systems and kits of the present invention may allow for the treatment of any vertebrae; for example, the expandable device can be applied to pelvic, lumbar, thoracic, or cervical vertebrae. Also, where the expandable device is emplaced between two vertebral discs or two vertebral bodies as part of a spinal corpectomy procedure, the kits and systems of the present invention may comprise emplacing a bone graft material 22 to promote fusion of the vertebral bodies.

For example, the material may comprise an autograft or allograft bone graft tissue (see e.g., Dick, Archives of Orthopaedic and Traumatic Surgery (1986), 105: 235-238; or Bhan et al, International Orthopaedics (SICOT) (1993) 17: 310-312). The bone graft tissue can be obtained using a Bone Graft Harvester, which is commercially available from SpineTech. Alternatively, the material may also comprise a granular bone material harvested from coral, e.g., PROOSTEON™ calcium carbonate granules, available from Interpore. The material for delivery to a bone can also comprise demineralized bone matrix suspended in glycerol (e.g., GRAFTON™ allograft material available from Osteotech), or SRS™ calcium phosphate cement available from Novian. The material for delivery to a bone can also be in sheet form, e.g., COLLAGRAFT™ material made from calcium carbonate powder and collagen from bovine bone. In an embodiment, the sheet may be rolled into a tube and loaded by hand into the access member. In one embodiment, KYPHX® HV-R™ bone cement, commercially available from Kyphon, Inc., may be used.

The kit may include an outer wrap and/or a case 88 to protect the components of the system from physical damage. In an embodiment, the kit may comprise a container for holding each of the parts under sterile conditions, or for transporting the parts from a first site to a second site. The kit may, in some embodiments, comprise a tray for holding the various parts in a secure position during sterilization and/or transport. The kit may also comprise a case to protect the components of the system from physical damage. In an embodiment, the outer wrap, tray, and/or casing may be made of materials commonly used in the art such as polyethylene and MYLAR™, to allow for visualization of the components in the kit. In an embodiment, the parts of the kit are arranged in an organized layout to facilitate use of the access member and other components of the kit for delivery of material to an internal body part. Also, labels identifying various parts of the kit may be included.

In an embodiment, the kit may comprise an inner seal, such as an inner wrap 90 that may be sealed by heat or vacuum, to prevent the components of the kit from being exposed to the outside environment. The inner seal may comprise a conventional peal-away seal to provide quick access to the components of the kit. Use of an inner and outer wrap may allow the kit to be prepared for imminent use by removing the outer wrap while leaving the inner wrap in place to ensure sterility of the kit components. The inner wrap may be made of materials such as TYVEK™ (DUPONT®), that is permeable to ethylene oxide (ETO) sterilizing gas. Sterilization may be by heat, pressure and/or sterilization gas as is known in the art. Also, the kit may include instructions for use by a physician or other trained personnel.

Each of the components used in the products, systems, and kits of the present invention may comprise a material that may be sterilized by either chemical treatment, high temperature, and/or high pressure, exposure to sterilizing gas, or a combination of sterilization treatments as is known in the art. Also, the components of the products, systems, and kits of the present invention may be disposable, or may be formulated to allow for cleaning, re-sterilization, and re-use.

### EXAMPLES

The present invention may be better understood by reference to the following nonlimiting examples.

### Example 1: Telescopic Corpectomy Device

Two telescopic corpectomy devices were made in accordance with alternate embodiments of the present invention. The devices were made of PEEK, and were able to expand from a compact length of 15 mm to 28 mm, and from 20 mm to 44 mm, respectively. The devices included a port at the base for attachment of a delivery catheter and teeth on both the bottom surface and the top surface. Both devices included 3 tiered cylinders that can be expanded upon introduction of PMMA or another type of bone filler material.

To make the devices, PEEK was machined into cylinders suitable for the housing (30 mm diameter), inner cylinders (about 24, 18, and 12 mm diameters), and the inner retainer ring (outer diameter equal to the inner diameter of the housing). The smallest cylinder was solid. Ribbing about 10 mm in width and 3-4 mm in depth, and wells about 12 mm in width and 3.5 to 4.5 mm in depth, were machined on the outside and inside of the cylinders, respectively. No wells were made in the smallest, solid cylinder. The PEEK used for the housing and internal cylinders was about 2.0 to 2.5 mm thick. Additionally, upper and lower retaining ledges, each extending about 2-3 mm from the wall of the cylinder were machined into each part.

The top and lower plates were machined with teeth, with each tooth being a pyramid of about 1 mm in height. Next, the unit was assembled by inserting the smallest cylinder through the bottom of the next largest cylinder. Thus, the third tier with its O-ring emplaced and held in place by slipping the O-ring under the ribbing, was inserted into the center of second tier with its O-ring emplaced, and then both tiers inserted into first tier with its O-ring emplaced. The assembly of tiered cylinders was then inserted into the housing. A retainer ring having an outer diameter the same as the inner diameter of the housing was made and heat sealed within the housing at a height of about 10 mm from the bottom of the housing. At this point, the upper surface was attached to the inner tier to close the unit. A screw was used to connect the upper surface to the innermost tier by inserting the screw into aperture on the upper surface of the tier.

### Example 2: Method of Posterior Corpectomy

A telescopic corpectomy device of the present invention was used during a corpectomy procedure on a cadaver. In this procedure, a vetebral body was accessed by a midline incision and posterior-lateral approach. Pedicle screws were placed, and a corpectomy of a vertebral body and removal of adjoining discs was conducted. The corpectomy device of the present invention was then delivered and inserted into the spinal void without retraction of the cord. In this procedure, the spikes on the endplate were retracted and so, did not come into contact with the cord. Next, the telescopic device was expanded inside of the spinal void by injection of cement into the device using a syringe. Once expanded, the endpieces of the device contacted the adjacent vetebral body endplates. At this point, the catheter was pulled away from the spine.

In some cases, the device is made of plastic and thus, is translucent to the x-rays. Thus, when the emplaced (and expanded) device is viewed by x-ray screening, the image revealed the upper screw 52 attaching the innermost member of the device to the contact element. Also visible was the bone filler cement 22, which is opaque to the x-rays, and the outline of the device (not shown).

The foregoing is considered as illustrative only of the principal of the invention. Since numerous modifications and changes will readily occur to those skilled in the art, it is not intended to limit the invention to the exact embodiments shown and described, and all suitable modifications and equivalents falling within the scope of the appended claims are deemed within the present inventive concept.

## Claims

1. An expandable device (2) for emplacement in a body part, the expandable device (2) comprising:
(a) a plurality of interlocking members (4, 6, 8, 10, 12) that can assume a first unexpanded configuration wherein a plurality (6, 8, 10, 12) of the interlocking members (4, 6, 8, 10, 12) are substantially contained within the body of a first (4) of the plurality of interlocking members (4, 6, 8, 10, 12), and a second expanded configuration wherein the interlocking members (4, 6, 8, 10, 12) are expanded such that each of the interlocking members (6, 8, 10, 12), which have been substantially contained within the body of the first (4) of the plurality of interlocking members, substantially emerges from at least one other interlocking member; and
(b) a contact element (15) that is attached to a surface (11) of a distal interlocking member (8, 10, 12) of the plurality of interlocking members (4, 6, 8, 10, 12) that is most distal to the first interlocking member (4) when the device (2) is in the expanded configuration, wherein the contact element (15) is contained within the first interlocking member (4) or provides a lid for the first interlocking member (4) when the device (2) is in the first unexpanded configuration, and wherein the contact element (15) is configured such that the surface (14) of the contact element (15) that is most distal to the first interlocking member (4) is suitable for engaging at least a portion of the body part, and wherein the surface (14) of the contact element (15) that is suitable for engaging at least a portion of the body part has a greater surface area and a greater circumference than the surface (11) of the interlocking member (8, 10, 12) to which the contact element (15) is attached.

2. The expandable device (2) of claim 1, wherein the interlocking members (4, 6, 8, 10, 12) expand along an axis (5) that is substantially perpendicular to the base of the first member (4).

3. The expandable device (2) of claim 1, wherein the surface (14) of the contact element (15) that is suitable for engaging at least a portion of the body part comprises a roughened surface.

4. The expandable device (2) of claim 1, wherein the first interlocking member (4) comprises an aperture (20) for insertion of a liquid or semi-solid material (22) into the interior of the first member (4).

5. The expandable device (2) of claim 4, wherein the material (22) comprises a bone filler material.

6. The expandable device (2) of claim 4, wherein the material (22) is used to expand the device (2).

7. The expandable device (2) of claim 4, wherein the material (22) is used to support the device (2) in the second expanded configuration.

8. The expandable device (2) of claim 1, wherein the plurality of interlocking members (4, 6, 8, 10, 12) are arranged concentrically around a first axis (5) such that the first interlocking member (4) is the outer-most member.

9. The expandable device (2) of claim 1, wherein each of the interlocking members (4, 6, 8, 10, 12) is sized to at least partly fit into another of the members.

10. The expandable device (2) of claim 1, wherein the device (2) expands by a telescopic mechanism such that a second interlocking member (6) expands out of a first member (4) and a third interlocking member (8) expands out of the second interlocking member (6) and any additional interlocking members expand in a similar sequential manner.

11. The expandable device (2) of claim 1, wherein each of the plurality of interlocking members (4, 6, 8, 10) comprise at least one retainer element (25-30) that functions to partly restrict movement of at least one other member away from the first member (4) during expansion of the device (2).

12. The expandable device (2) of claim 1, wherein the expandable device (2) is capable of providing support to a spine of a subject when the device (2) is implanted in the subject's spine during a corpectomy.

13. The expandable device (2) of claim 1, wherein the expandable device (2) is suitable for use to replace a vertebral body and/or an intervertebral disc in a human spine.

14. The expandable device (2) of claim 1, wherein the device (2) comprises a size prior to expansion that enables the device (2) to be inserted into the spine during a posterior corpectomy.

15. A kit comprising:
(a) an expandable device (2) according to any of the preceding claims; and
(b) an access member (21, 74) configured to be attached to the device (2) and suitable for using to insert the device (2) into the body part.

## Patentansprüche

1. Eine expandierbare Vorrichtung (2) zum Platzieren in einem Körperteil, wobei die expandierbare Vorrichtung (2) aufweist:
(a) eine Mehrzahl von Verriegelungselementen (4, 6, 8, 10, 12), die annehmen können: eine erste Nicht-Expansionskonfiguration, wobei eine Mehrzahl (6, 8, 10, 12) der Verriegelungselemente (4, 6, 8, 10, 12) im Wesentlichen in dem Körper eines ersten (4) aus der Mehrzahl von Verriegelungselementen (4, 6, 8, 10, 12) enthalten ist, und eine zweite Expansionskonfiguration, wobei die Verriegelungselemente (4, 6, 8, 10, 12) derart expandiert sind, dass jedes der Verriegelungselemente (6, 8, 10, 12), die im Wesentlichen im Körper des ersten (4) aus der Mehrzahl von Verriegelungselementen enthalten waren, im Wesentlichen aus mindestens einem anderen Verriegelungselement herauskommt, und
(b) ein Kontaktelement (15), das an einer Fläche (11) eines distalen Verriegelungselements (8, 10, 12) der Mehrzahl von Verriegelungselementen (4, 6, 8, 10, 12) angebracht ist, das am distalsten zu dem ersten Verriegelungselement (4) ist, wenn sich die Vorrichtung (2) in der Expansionskonfiguration befindet, wobei das Kontaktelement (15) in dem ersten Verriegelungselement (4) enthalten ist oder einen Deckel für das erste Verriegelungselement (4) bereitstellt, wenn sich die Vorrichtung (2) in der ersten Nicht-Expansionskonfiguration befindet, und wobei das Kontaktelement (15) derart eingerichtet ist, dass die Fläche (14) des Kontaktelements (15), die am distalsten zu dem ersten Verriegelungselement (4) ist, für einen Eingriff mit zumindest einem Abschnitt des Körperteils geeignet ist, und wobei die Fläche (14) des Kontaktelements (15), die für einen Eingriff mit zumindest einem Abschnitt des Körperteils geeignet ist, eine größere Flächenausdehnung und einen größeren Umfang als die Fläche (11) des Verriegelungselements (8, 10, 12) hat, an der das Kontaktelement (15) angebracht ist.

2. Die expandierbare Vorrichtung (2) gemäß Anspruch 1, wobei die Verriegelungselemente (4, 6, 8, 10, 12) entlang einer Achse (5) expandieren, die im Wesentlichen senkrecht zu der Basis des ersten Elements (4) ist.

3. Die expandierbare Vorrichtung (2) gemäß Anspruch 1, wobei die Fläche (14) des Kontaktelements (15), die für einen Eingriff mit zumindest einem Abschnitt des Körperteils geeignet ist, eine aufgeraute Fläche aufweist.

4. Die expandierbare Vorrichtung (2) gemäß Anspruch 1, wobei das erste Verriegelungselement (4) eine Öffnung (20) zum Einführen eines flüssigen oder halbfesten Materials (22) in das Innere des ersten Elements (4) aufweist.

5. Die expandierbare Vorrichtung (2) gemäß Anspruch 4, wobei das Material (22) ein Knochenfüllmaterial aufweist.

6. Die expandierbare Vorrichtung (2) gemäß Anspruch 4, wobei das Material (22) zum Expandieren der Vorrichtung (2) verwendet wird.

7. Die expandierbare Vorrichtung (2) gemäß Anspruch 4, wobei das Material (22) zum Abstützen der Vorrichtung (2) in der zweiten Expansionskonfiguration verwendet wird.

8. Die expandierbare Vorrichtung (2) gemäß Anspruch 1, wobei die Mehrzahl von Verriegelungselementen (4, 6, 8, 10, 12) konzentrisch um eine erste Achse (5) angeordnet ist, so dass das erste Verriegelungselement (4) das äußerste Element ist.

9. Die expandierbare Vorrichtung (2) gemäß Anspruch 1, wobei jedes der Verriegelungselemente (4, 6, 8, 10, 12) bemessen ist, um zumindest teilweise in ein anderes der Elemente zu passen.

10. Die expandierbare Vorrichtung (2) gemäß Anspruch 1, wobei die Vorrichtung (2) durch einen Teleskopmechanismus expandiert, so dass ein zweites Verriegelungselement (6) aus einem ersten Element (4) heraus expandiert und ein drittes Verriegelungselement (8) aus dem zweiten Verriegelungselement (6) heraus expandiert und irgendwelche zusätzlichen Verriegelungselemente auf eine ähnliche sequentielle Weise expandieren.

11. Die expandierbare Vorrichtung (2) gemäß Anspruch 1,
wobei jedes aus der Mehrzahl von Verriegelungselementen (4, 6, 8, 10) mindestens ein Halteelement (25 bis 30) aufweist, das funktioniert, um die Bewegung von mindestens einem anderen Element weg von dem ersten Element (4) während des Expandierens der Vorrichtung (2) teilweise zu beschränken.

12. Die expandierbare Vorrichtung (2) gemäß Anspruch 1,
wobei die expandierbare Vorrichtung (2) in der Lage ist, einer Wirbelsäule einer Person Unterstützung bereitzustellen, wenn die Vorrichtung (2) während einer Korpektomie in die Wirbelsäule der Person implantiert wird.

13. Die expandierbare Vorrichtung (2) gemäß Anspruch 1, wobei die expandierbare Vorrichtung (2) für eine Verwendung geeignet ist, um einen Wirbelkörper und/oder eine Bandscheibe in einer menschlichen Wirbelsäule zu ersetzen.

14. Die expandierbare Vorrichtung (2) gemäß Anspruch 1, wobei die Vorrichtung (2) vor dem Expandieren eine Größe hat, die ermöglicht, dass die Vorrichtung (2) während einer posterioren Korpektomie in die Wirbelsäule eingesetzt wird.

15. Ein Kit, aufweisend:
(a) eine expandierbare Vorrichtung (2) gemäß irgendeinem der vorhergehenden Ansprüche, und
(b) ein Zugangselement (21, 74), das eingerichtet ist, um an der Vorrichtung (2) angebracht zu sein, und geeignet ist, um zum Einsetzen der Vorrichtung (2) in den Körperteil verwendet zu werden.

## Revendications

1. Dispositif extensible (2) destiné à être placé dans une partie corporelle, le dispositif extensible (2) comprenant :
(a) une pluralité d'éléments d'emboîtement (4, 6, 8, 10, 12) qui peuvent adopter une première configuration non étendue dans laquelle une pluralité (6, 8, 10, 12) des éléments d'emboîtement (4, 6, 8, 10, 12) sont sensiblement contenus à l'intérieur du corps d'un premier (4) de la pluralité d'éléments d'emboîtement (4, 6, 8, 10, 12), et une deuxième configuration étendue dans laquelle les éléments d'emboîtement (4, 6, 8, 10, 12) sont étendus de telle sorte que chacun des éléments d'emboîtement (6, 8, 10, 12), qui ont été sensiblement contenus à l'intérieur du corps du premier (4) de la pluralité d'éléments d'emboîtement, émerge sensiblement d'au moins un autre élément d'emboîtement ; et
(b) un élément de contact (15) qui est attaché à une surface (11) d'un élément d'emboîtement distal (8, 10, 12) de la pluralité d'éléments d'emboîtement (4, 6, 8, 10, 12) qui est le plus éloigné du premier élément d'emboîtement (4) lorsque le dispositif (2) est dans la configuration étendue, dans lequel l'élément de contact (15) est contenu à l'intérieur du premier élément d'emboîtement (4) ou fournit un couvercle pour le premier élément d'emboîtement (4) lorsque le dispositif (2) est dans la première configuration non étendue, et dans lequel l'élément de contact (15) est configuré de telle sorte que la surface (14) de l'élément de contact (15) qui est le plus éloigné du premier élément d'emboîtement (4) soit appropriée pour venir en prise avec au moins une section de la partie corporelle, et dans lequel la surface (14) de l'élément de contact (15) qui est appropriée pour venir en prise avec au moins une section de la partie corporelle a une plus grande aire de surface et une plus grande circonférence que la surface (11) de l'élément d'emboîtement (8, 10, 12) à laquelle l'élément de contact (15) est attaché.

2. Dispositif extensible (2) selon la revendication 1, dans lequel les éléments d'emboîtement (4, 6, 8, 10, 12) s'étendent le long d'un axe (5) qui est sensiblement perpendiculaire à la base du premier élément (4).

3. Dispositif extensible (2) selon la revendication 1, dans lequel la surface (14) de l'élément de contact (15) qui est appropriée pour venir en prise avec au moins une partie de la partie corporelle comprend une surface rugosifiée.

4. Dispositif extensible (2) selon la revendication 1, dans lequel le premier élément d'emboîtement (4) comprend une ouverture (20) pour l'insertion d'un matériau liquide ou semi-solide (22) jusqu'à l'intérieur du premier élément (4).

5. Dispositif extensible (2) selon la revendication 4, dans lequel le matériau (22) comprend un matériau de remplissage d'os.

6. Dispositif extensible (2) selon la revendication 4, dans lequel le matériau (22) est utilisé pour étendre le dispositif (2).

7. Dispositif extensible (2) selon la revendication 4, dans lequel le matériau (22) est utilisé pour porter le dispositif (2) dans la deuxième configuration étendue.

8. Dispositif extensible (2) selon la revendication 1, dans lequel la pluralité d'éléments d'emboîtement (4, 6, 8, 10, 12) sont agencés de manière concentrique autour d'un premier axe (5) de telle sorte que le premier élément d'emboîtement (4) soit l'élément le plus à l'extérieur.

9. Dispositif extensible (2) selon la revendication 1, dans lequel chacun des éléments d'emboîtement (4, 6, 8, 10, 12) est dimensionné pour s'insérer au moins partiellement dans un autre des éléments.

10. Dispositif extensible (2) selon la revendication 1, dans lequel le dispositif (2) s'étend par un mécanisme télescopique de telle sorte qu'un deuxième élément d'emboîtement (6) s'étend hors d'un premier élément (4) et qu'un troisième élément d'emboîtement (8) s'étend hors du deuxième élément d'emboîtement (6) et que tous les éléments d'emboîtement supplémentaires s'étendent d'une manière séquentielle similaire.

11. Dispositif extensible (2) selon la revendication 1, dans lequel chacun de la pluralité d'éléments d'emboîtement (4, 6, 8, 10) comprend au moins un élément de retenue (25 à 30) qui fonctionne pour limiter partiellement le mouvement d'au moins un autre élément éloigné du premier élément (4) pendant l'extension du dispositif (2).

12. Dispositif extensible (2) selon la revendication 1, dans lequel le dispositif extensible (2) est capable de fournir un support à la colonne vertébrale d'un sujet lorsque le dispositif (2) est implanté dans la colonne vertébrale du sujet pendant une corporectomie. ¹

13. Dispositif extensible (2) selon la revendication 1, dans lequel le dispositif extensible (2) est approprié pour être utilisé pour remplacer un corps vertébral et/ou un disque intervertébral dans une colonne vertébrale humaine.

14. Dispositif extensible (2) selon la revendication 1, dans lequel le dispositif (2) présente, avant son extension, une taille qui permet au dispositif (2) d'être inséré jusque dans la colonne vertébrale pendant une corporectomie postérieure.

15. Kit comprenant :
(a) un dispositif extensible (2) selon l'une quelconque des revendications précédentes ; et
(b) un élément d'accès (21, 74) configuré pour être attaché au dispositif (2) et approprié pour être utilisé pour insérer le dispositif (2) jusque dans la partie corporelle.
